# EUROPEAN PATENT SPECIFICATION

(11) **EP 4 015 636 B1**
(45) Date of publication and mention of the grant of the patent: **20.11.2024**
(21) Application number: 21209636.6
(22) Date of filing: 21.05.2008
(51) Int. Cl.: C12N 15/117, C07H 21/00, A61K 31/7105, A61K 31/7115, A61K 31/713

(54) **5' TRIPHOSPHATE OLIGONUCLEOTIDE WITH BLUNT END AND USES THEREOF**
5' -TRIPHOSPHAT-OLIGONUKLEOTID MIT STUMPFEM ENDE, UND DESSEN VERWENDUNGEN
OLIGONUCLÉOTIDE DE TRIPHOSPHATE 5' AVEC EXTRÉMITÉ FRANCHE ET UTILISATIONS ASSOCIÉES

(43) Date of publication of application: 22.06.2022
(62) Divisional of application: 19174691.6
(73) Proprietor: Rheinische Friedrich-Wilhelms-Universität Bonn, 53113 Bonn (DE)
(72) Inventor: HARTMANN, Gunther, 53127 Bonn (DE); SCHLEE, Martin, 53127 Bonn (DE)
(74) Representative: Wichmann, Hendrik

(56) References cited:
- WO-A2-2007/038788
- WO-A2-2008/017473
- PAOLO FRUSCOLONI ET AL: "Exonucleolytic degradation of double-stranded RNA by an activity in Xenopus laevis germinal vesicles", PROCEEDINGS OF THE NATIONAL ACADEMY OF SCIENCES (PNAS), US, vol. 100, no. 4, 18 February 2003 (2003-02-18), pages 1639 - 1644, XP008150373, ISSN: 0027-8424, [retrieved on 20030210], DOI: 10.1073/PNAS.252777499
- BOWIE ET AL: "RIG-I: tri-ing to discriminate between self and non-self RNA", TRENDS IN IMMUNOLOGY, ELSEVIER, RAHWAY, NJ, US, vol. 28, no. 4, 27 March 2007 (2007-03-27), pages 147 - 150, XP022002743, ISSN: 1471-4906, DOI: 10.1016/J.IT.2007.02.002
- SCHLEE M ET AL: "siRNA and isRNA: two edges of one sword", MOLECULAR THERAPY, ACADEMIC PRESS, SAN DIEGO, CA, US, vol. 14, no. 4, 2006, pages 463 - 470, XP002416372, ISSN: 1525-0016
- SIOUD ET AL: "RNA interference and innate immunity", ADVANCED DRUG DELIVERY REVIEWS, ELSEVIER BV, AMSTERDAM, NL, vol. 59, no. 2-3, 30 March 2007 (2007-03-30), pages 153 - 163, XP022087322, ISSN: 0169-409X
- KAO ET AL: "DE NOVO INITIATION OF VIRAL RNA-DEPENDENT RNA SYNTHESIS", VIROLOGY, ELSEVIER, AMSTERDAM, NL, vol. 287, no. 2, September 2001 (2001-09-01), pages 251 - 260, XP003035917, ISSN: 0042-6822
- SCHLEE M ET AL: "BEYOND DOUBLE-STRANDED RNA-TYPE I IFN INDUCTION BY 3PRNA AND OTHER VIRAL NUCLEICACIDS", TOP. MICROBIOL. IMMUNOL, XX, XX, vol. 316, 2007, pages 207 - 230, XP003036141
- ROHAYEM JACQUES ET AL: "Protein-primed and de novo initiation of RNA synthesis by norovirus 3Dpol", JOURNAL OF VIROLOGY, THE AMERICAN SOCIETY FOR MICROBIOLOGY, US, vol. 80, no. 14, July 2006 (2006-07-01), pages 7060 - 7069, XP002544507, ISSN: 0022-538X, DOI: 10.1128/JVI.02195-05
- FULLERTON STEPHEN W B ET AL: "Structural and functional characterization of sapovirus RNA-dependent RNA polymerase", JOURNAL OF VIROLOGY, THE AMERICAN SOCIETY FOR MICROBIOLOGY, US, vol. 81, no. 4, February 2007 (2007-02-01), pages 1858 - 1871, XP002544505, ISSN: 0022-538X, DOI: 10.1128/JVI.01462-06
- I. D. VILFAN ET AL: "An RNA toolbox for single-molecule force spectroscopy studies", NUCLEIC ACIDS RESEARCH, vol. 35, no. 19, January 2007 (2007-01-01), pages 6625 - 6639, XP055301559, ISSN: 0305-1048, DOI: 10.1093/nar/gkm585

## Description

### FIELD OF THE INVENTION

The present invention relates to the field of immunotherapy and drug discovery. The present invention provides a oligonucleotide which is capable of activating RIG-I and inducing an antiviral IFN response in cells expressing RIG-I. The present invention further provides the use of said oligonucleotide for inducing an anti-viral IFN response in vitro and in vivo. The present invention additionally provides the use of said oligonucleotide for preventing and/or treating diseases or conditions such as infections, tumors/cancers, and immune disorders, as further defined in the claims.

### BACKGROUND OF THE INVENTION

The presence of viral nucleic acids represents a danger signal for the immune system which initiates an anti-viral response to impede viral replication and eliminate the invading pathogen¹. Interferon (IFN) response is a major component of the anti-viral response and comprises the production of type I IFNs, IFN-α and IFN-β. An anti-viral response also comprises the production of various other cytokines, such as IL-12, which promote innate and adaptive immunity¹.

In order to detect foreign nucleic acids, immune cells are equipped with a set of pattern recognition receptors (PRR) which act at the frontline of the recognition process and which can be grouped into two major classes: the Toll-like receptors and the RNA helicases.

Members of the Toll-like receptor (TLR) family have been implicated in the detection of long dsRNA (TLR3) ², ssRNA (TLR7 and 8) ^{3,4}, short dsRNA (TLR7) ⁵ and CpG DNA (TLR9) ⁶. The TLRs reside primarily in the endosomal membranes of the immune cells ^{7,8} and recognize viral nucleic acids that have been taken up by the immune cells into the endosomal compartments 9

RNA helicases, such as RIG-I, MDA-5 and LGP-2, have been implicated in the detection of viral RNA ^{10,11}. In contrast to the TLRs, the RNA helicases are cytosolic and are expressed in a wide spectrum of cell types, including immune cells and non-immune cells, such as fibroblasts and epithelial cells ¹². Therefore, not only immune cells, but also non-immune cells which express one or more of the RNA helicase(s), are capable of detecting and responding to viral RNA.

Both the TLR and the RNA helicase systems co-operate to optimally detect viral RNA.

Given the abundance of host RNA present in the cytoplasm, it is an intricate task to specifically and reliably detect virus-derived RNA. Maximal sensitivity together with a high degree of specificity for "non-self" are required. Two major mechanisms appear to be in place in vertebrate cells to distinguish "non-self" from "self" nucleic acids via a protein receptor-based recognition system: (1) the detection of a pathogen-specific compartmentalization, and (2) the detection of a pathogen-specific molecular signature or motif.

Endosomal RNA is recognized by the TLRs as "non-self" ⁴. Notably, host RNA can acquire the ability to stimulate an IFN response via the activation of TLRs under certain pathological situations ¹³.

Furthermore, there exist structural motifs or molecular signatures that allow the pattern recognition receptors (PRRs) to determine the origin of an RNA. For example, long dsRNA has been proposed to stimulate an IFN response via TLR3 ², RIG-I ¹¹ and MDA-5 ¹⁴. The present inventors recently identified the 5' triphosphate moiety of viral RNA transcripts as the ligand for RIG-I ^{15, 19}. Furthermore, it has been shown in the art that exonucleolytic degradation of double-stranded RNA by an activity in Xenopus laevis germinal vesicles occurs³¹. Even though nascent nuclear endogenous host RNA transcripts initially also contain a 5' triphosphate, several nuclear post-transcriptional modifications, including 5' capping, endonucleolytic cleavage, and base and backbone modifications, of the nascent RNA transcripts lead to mature cytoplasmic RNAs which are ignored by RIG-I. In addition, blunt-ended short dsRNA without any 5' phosphate group ¹⁶ or with 5' monophosphate ²⁴ has also been postulated to stimulate RIG-I. A clearly defined molecular signature has not yet been reported for the ssRNA-sensing TLRs, TLR7 and TLR8. However, the fact that certain sequence motifs are better recognized than others by the TLRs suggests that the nucleotide composition of the RNA, on top of endosomal localization, may represent basis for discriminating between "self' and "non-self' by the TLRs ^{3-5, 10, 11, 17, 18}.

Despite its pivotal role in anti-viral defense, the mechanism of viral RNA recognition by RIG-I is not yet fully elucidated. Therefore, there is a need in the art to better understand the recognition of viral and/or other non-self RNA by RIG-I. Furthermore, given the efficacy of IFN-α in various clinical applications, there is a need in the art to provide alternative agents for inducing IFN-α production in vitro and in vivo.

It is therefore an object of the present invention to further identify the structural motifs or molecular signatures of an RNA molecule which are recognized by RIG-I. It is another object of the present invention to prepare RNA molecules which are capable of activating RIG-I and inducing an anti-viral, in particular, an IFN, response in cells expressing RIG-I. It is a further object of the present invention to use said RNA molecules for inducing an anti-viral, in particular, an IFN, response in vitro and in vivo. It is an ultimate object of the present invention to use said RNA molecules for preventing and/or treating diseases or conditions which would benefit from an anti-viral, in particular, an IFN, response, such as infections, tumors/cancers, and immune disorders.

### SUMMARY OF THE INVENTION

The present invention provides an essentially homogenous population of a chemically synthesized oligonucleotide, which is capable of activating RIG-I and inducing a type I IFN response, wherein said essentially homogenous population means that at least 85 % of the oligonucleotides have the same chemical identity, including the same nucleotide sequence, backbone, modifications, length, and end structures, wherein the oligonucleotide has two blunt ends, wherein the blunt ends are ends of a fully double-stranded section, and wherein the fully double-stranded section is at least 21 and at most 30 base pairs in length, and the double-stranded oligonucleotide has one blunt end bearing a 5' triphosphate attached to the most 5' ribonucleotide and a second blunt end which does not bear a 5' triphosphate, wherein the most 5' ribonucleotide with the triphosphate attached to is G, U or C, wherein the oligonucleotide comprises at least 1, preferably at least 3, more preferably at least 6 ribonucleotide(s) at the 5' end at the blunt end bearing the 5' triphosphate, and wherein the oligonucleotide is free of modifications selected from the group consisting of pseudouridine, 2-thiouridine, 2'Fluorine-dNTP and 2-O-methylated NTP, or wherein the oligonucleotide contains modified nucleotides that do not compromise the type I IFN-inducing activity, wherein the 5' triphosphate is free of any cap structure.

In one embodiment, the oligonucleotide comprises at least one inosine.

In some embodiments, the most 5' ribonucleotide with the triphosphate attached to is selected from G and U, preferably G.

It is disclosed but not claimed that the sequence of the first 4 ribonucleotides at the 5' end is selected from: AAGU, AAAG, AUGG, AUUA, AACG, AUGA, AGUU, AUUG, AACA, AGAA, AGCA, AACU, AUCG, AGGA, AUCA, AUGC, AGUA, AAGC, AACC, AGGU, AAAC, AUGU, ACUG, ACGA, ACAG, AAGG, ACAU, ACGC, AAAU, ACGG, AUUC, AGUG, ACAA, AUCC, AGUC, wherein the sequence is in the 5'->3' direction.

The oligonucleotide is free of modifications selected pseudouridine, 2-thiouridine, 2'-Fluorine-dNTP, 2'-O-methylated NTP, in particular 2'-fluorine-dCTP, 2'-fluorine-dUTP, 2'-O-methylated CTP, 2'-O-methylated UTP.

In a further embodiment, the oligonucleotide comprises at least one structural motif recognized by at least one of TLR3, TLR7, TLR8 and TLR9.

In an additional embodiment, the oligonucleotide has gene-silencing activity.

The present invention provides a pharmaceutical composition comprising at least one oligonucleotide preparation of the present invention and a pharmaceutically acceptable carrier, as further defined in the claims.

In one embodiment, the pharmaceutical composition further comprises at least one agent selected from an immunostimulatory agent, an anti-viral agent, an anti-bacterial agent, an anti-tumor agent, retinoic acid, IFN-α, and IFN-β.

The present invention further provides the use of at least one oligonucleotide preparation of the present invention for the preparation of a composition for inducing type I IFN production, as further defined in the claims.

The present invention also provides the use of at least one oligonucleotide preparation of the present invention for the preparation of a composition for preventing and/or treating a disease or condition selected from an infection, a tumor, and an immune disorder, as further defined in the claims.

In one embodiment, the oligonucleotide preparation is used in combination with at least one agent selected from an immunostimulatory agent, an anti-viral agent, an anti-bacterial agent, an anti-tumor agent, retinoic acid, IFN-α, and IFN-β.

In another embodiment, the composition is prepared for administration in combination with at least one treatment selected from a prophylactic and/or a therapeutic treatment of an infection, a tumor, and an immune disorder.

The present invention additionally provides an *in vitro* method for inducing type I IFN production in a cell, comprising the steps of:
(a) mixing at least oligonucleotide preparation of the present invention with a complexation agent; and
(b) contacting a cell with the mixture of (a), wherein the cell expresses RIG-I.

### BRIEF DESCRIPTION OF THE DRAWINGS

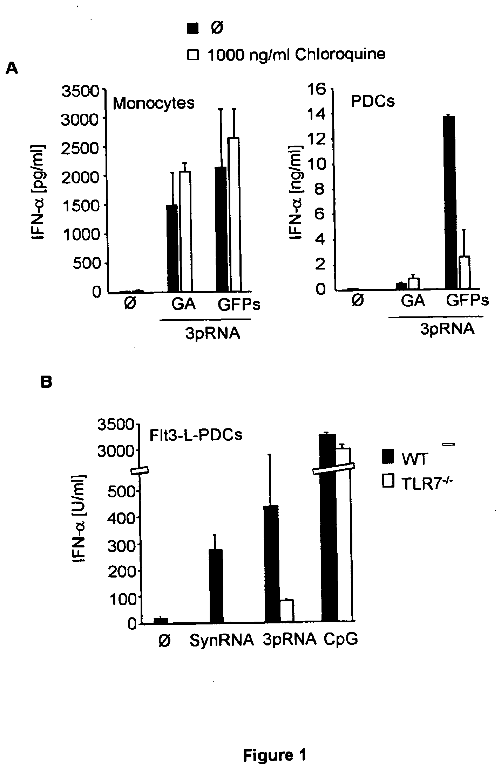

**Figure 1****.** Recognition of 3pRNA occurs through differential pathways in monocytes and PDCs. (A) Monocytes and PDCs were pre-incubated with 1000 ng/ml chloroquine (white bars) or left untreated (black bars). After 30min cells were stimulated with 3pRNA GA or 3pRNA GFPs complexed with lipofectamine (as indicated) or control (no nucleic acid). Supernatants were collected 20h post stimulation and IFN-α production was determined by ELISA. The results are representative for two experiments and mean ± SEM is shown. (B) Sorted PDCs from Flt3-L-induced bone marrow cultures of wild-type (WT; black bars) and TLR7-deficient mice (TLR7^{-/-}; white bars) were transfected with 200ng of SynRNA, 3pRNA and CpG ODN2216 (3 µg/ml). After 24h, IFN-α production was determined by ELISA. Data are expressed as the mean ± SEM of two independent experiments.

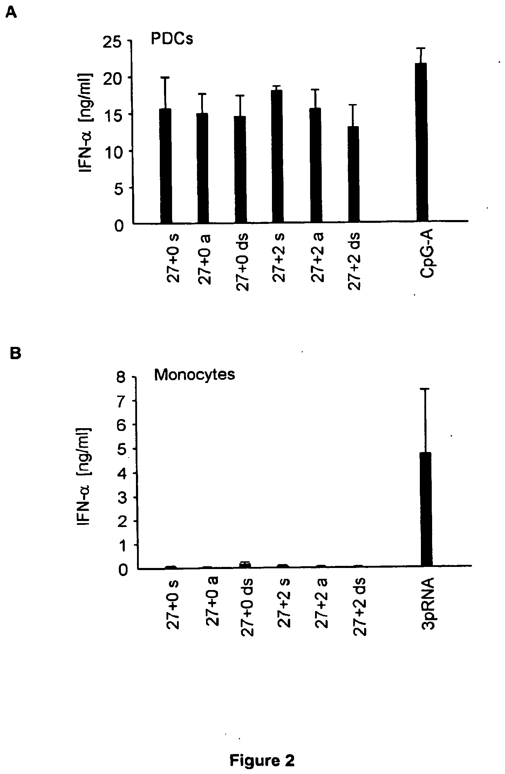

**Figure 2****.** Blunt end synthetic dsRNA is a poor inducer of IFN-α in monocytes. (A-B) Synthetic ssRNA and dsRNA with (27+2) or without (27+0) 2-nt overhangs were transfected into PDCs and monocytes in complex with lipofectamine 2000. CpG ODN 2216 (3 µg/ml) and 3pRNA GFPs (200ng) were used as a positive control stimulus for TLR and RIG-I, respectively. The levels of IFN-α production were analyzed by ELISA 24h after stimulation and are depicted as mean ± SEM of two independent experiments.

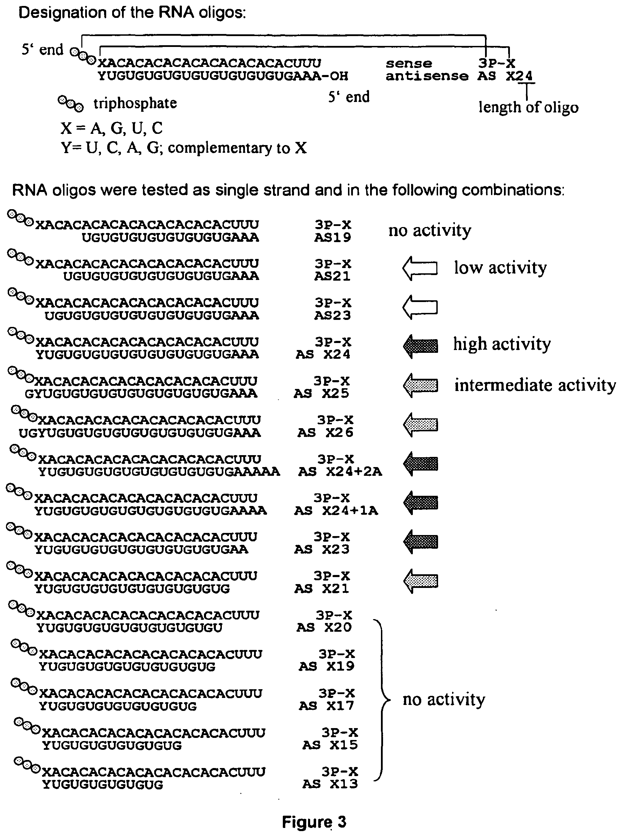

**Figure 3****.** Experimental design: synthetic RNA oligonucleotides used to test the effect of end structure on the immunostimulatory activity of double-stranded 3pRNA.

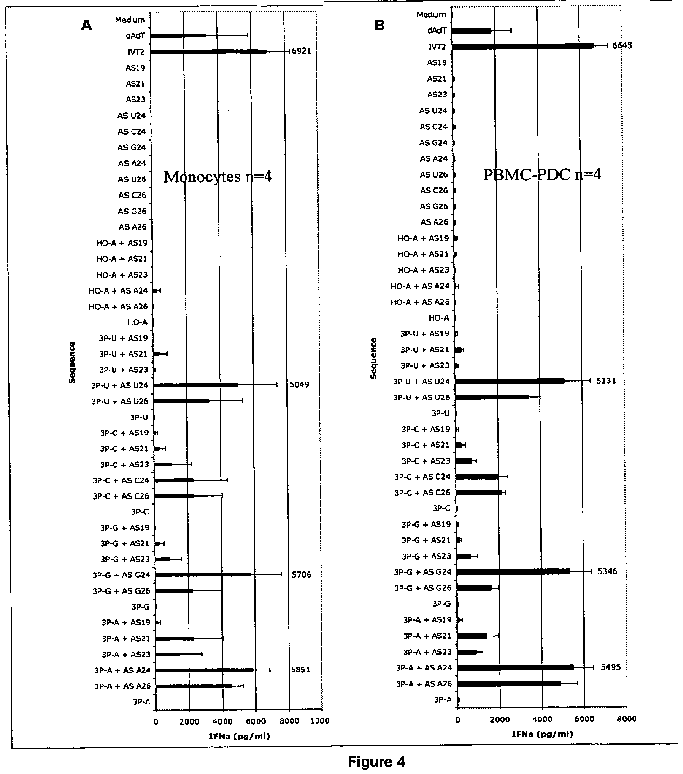

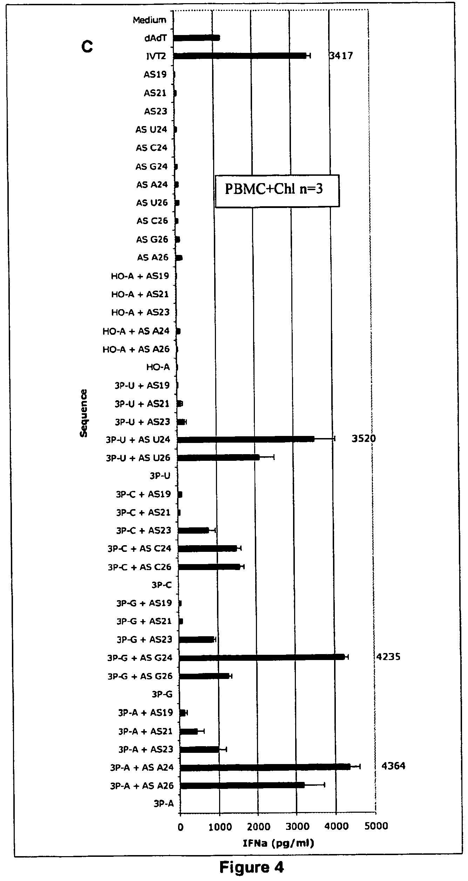

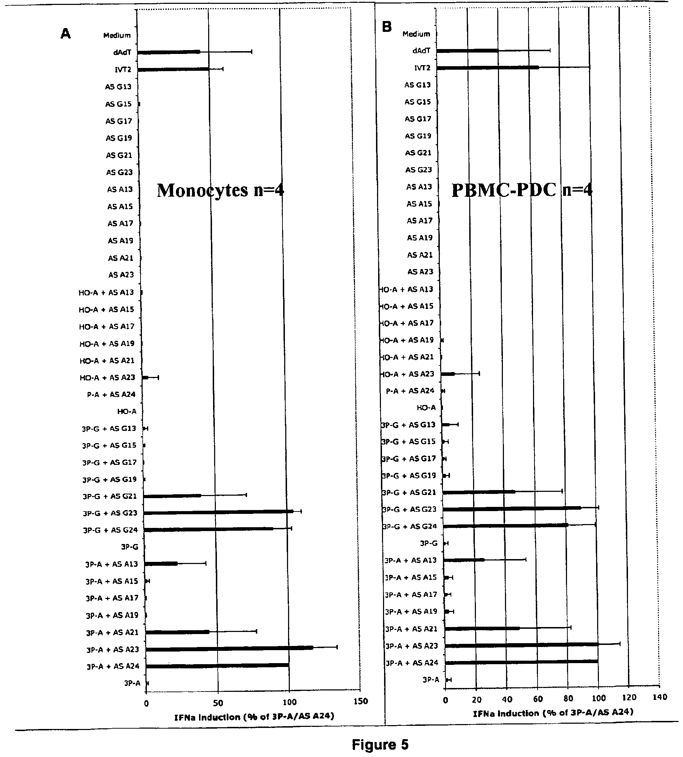

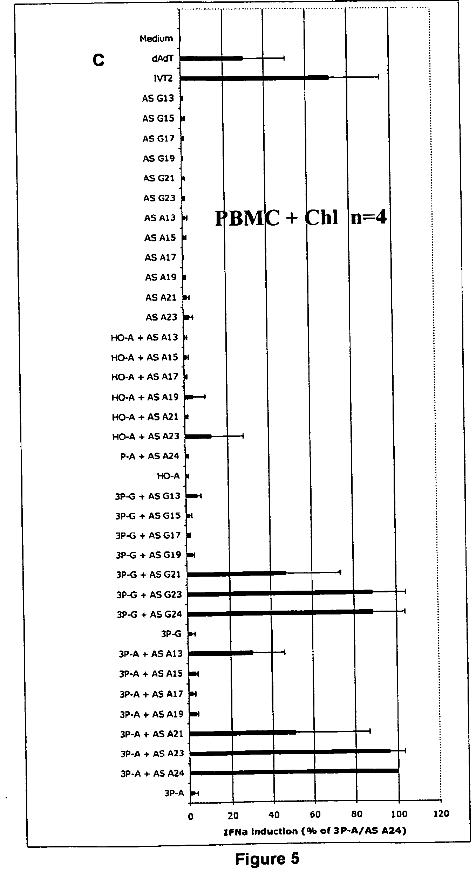

**Figures 4-6****.** Blunt end at the end bearing the 5' triphosphate augments the immunostimulatory activity of synthetic double-stranded 3pRNA oligonucleotides. Purified monocytes, PDC-depleted PBMCs and PBMCs pre-treated with chloroquine were transfected with the indicated single-stranded or double-stranded synthetic RNA oligonucleotide. IFN-α production was analyzed 24 hours after stimulation. Data from three or four independent donors were summarized and are depicted as mean values ± SEM.

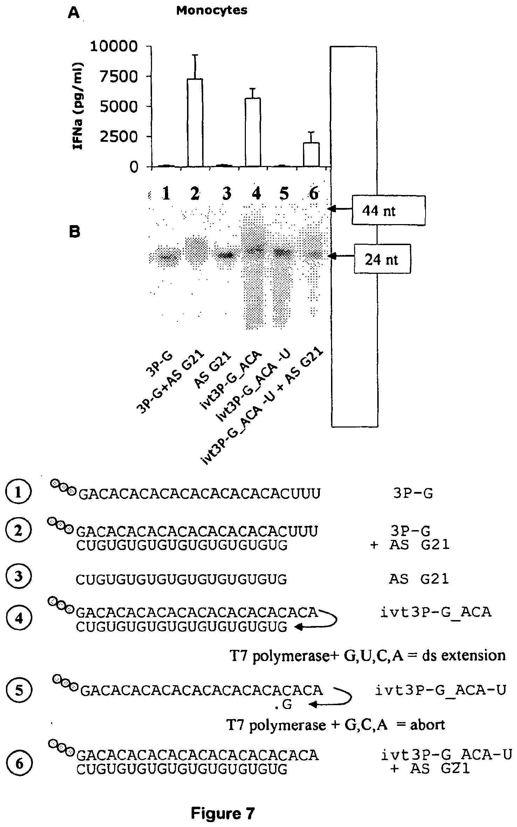

**Figures 7****.** Single-stranded RNA oligonucleotide can be obtained by in vitro transcription. (A) In vitro transcribed, synthetic or mixed double-stranded or single-stranded oligonucleotides were transfected into purified monocytes. IFN-α production was analyzed 24 hours after stimulation. (B) Urea polyacrylamide gel electrophoresis. (C) Sequence of the oligonucleotides tested.

### DETAILED DESCRIPTION OF THE INVENTION

Initially, it was reported that *in vitro* transcribed siRNAs (small-interfering RNA) which bear 5' triphosphate, but not synthetic siRNAs which bear 5' OH, stimulated the production of type I IFN from selected cell lines ^{20, 21} . However, the molecular mechanism which led to the induction of type I IFN was not known.

Subsequently, it was reported that *in vitro* transcribed long dsRNA (equal or longer than 50bp) was detected by RIG-I ¹⁰.

Almost at the same time, it was reported that synthetic blunt-ended short dsRNAs 21-27bp in length and without any 5' phosphate group were ligands for RIG-I ¹⁶. Furthermore, it was reported that 2-nucleotide 3' overhangs, and to a lesser extend, 5' overhangs, inactivated RIG-I ¹⁶. It was postulated that blunt end was the molecular signature recognized by RIG-I.

Shortly thereafter, in vitro transcribed short ssRNA and dsRNA bearing 5' triphosphate were identified as the ligands for RIG-I ^{15, 19}. Furthermore, it was shown that *in vitro* transcribed short dsRNAs having the same sequences as those used in Marques TJ et al. (2006) ¹⁶ stimulated IFN-α production in purified primary human monocytes to the same degree, regardless whether the dsRNA had blunt ends or 3' overhangs ¹⁵. In other words, in the presence of 5' triphosphate, the end structure of a dsRNA oligonucleotide did not affect the IFN-α-inducing activity of the oligonucleotide; the presence of 3' overhangs did not inactivate RIG-I. This finding confirms the notion that 5' triphosphate is a molecular signature recognized by and activates RIG-I ^{15, 19} and suggests that blunt end is not a molecular signature that activates RIG-I ¹⁶.

At the same time, it was reported that single-stranded genomic RNA from influenza A which bears 5' phosphates was recognized by RIG-I ²⁵.

More recently, it was reported that the C-terminal regulatory domain RD of RIG-I was responsible for the recognition of the 5' triphosphate on in vitro transcribed ssRNA ligands ²⁶.

Almost at the same time, it was reported that dsRNA 25-nucleotide long with a 5' monophosphate was also a ligand for RIG-I ²⁴. Furthermore, ssRNA bearing 5' triphosphate was confirmed to be a ligand for RIG-I ²⁴. Moreover, it was reported that a blunt-ended dsRNA bearing a 5' monophosphate was more potent at inducing an IFN response than that having 3' overhangs ²⁴. However, counterintuitively, it was also reported that 5' monophosphate in dsRNA was not required for interaction with the C-terminal domain of RIG-I whereas 5' triphosphate did interact with the C-terminal domain. In addition, it was reported that the ability of a dsRNA oligonucleotide to induce an IFN response was inversely correlated with the unwinding activity by the helicase domain, which contradicts earlier report that blunt end was not only required for the induction of an IFN response, but also for helicase activity ¹⁶.

Also very recently, it was reported that the presence of 2 or 3 G's at the 5' end of shRNAs generated by *in vitro* transcription obliterated the ability of the 5' triphosphate-bearing RNA to induce IFN-β production via the RIG-I pathway ³⁰. Notably, the shRNAs bearing two 5' G's, which had two G-U wobble base pairs at the end, i.e., a blunt end, were completely inactive in inducing an IFN response.

In summary, a number of structurally different RNA molecules have been reported to be the ligand for RIG-I and different molecular signatures have been postulated to be recognized by RIG-I. The data in the prior art were inconsistent and at times conflicting with regard to the molecular signatures that were important for activating RIG-I. In other words, there was no consensus in the prior art with regard to the critical molecular signature(s) for RIG-I recognition and activation.

Even though various immune cell types, such as monocytes, plasmacytoid dendritric cells (PDCs), and myeloid dendritic cells (MDCs), are capable of producing IFN-α in response to stimulation by double-stranded RNA oligonucleotides bearing 5' triphosphate (hereinafter, "3pRNA"; unpublished data from the present inventors), the recognition of such double-stranded 3pRNA oligonucleotides appears to be mediated by different receptors in different cell types. Whereas PDCs appear to utilize TLR7 primarily, monocytes appear to utilize RIG-I primarily (Example 1; Figure 1). In fact, PDC is the only cell type that produces a significant amount of IFN-α upon stimulation with an appropriate TLR ligand. In contrast, other immune cells, such as myeloid cells, produce cytokines other than IFN-α in response to stimulation by TLR ligands. Therefore, monocytes (without contaminating functional PDCs) are ideal for studying the mechanism of ligand recognition by and the activation of RIG-I, with IFN-α production as the readout.

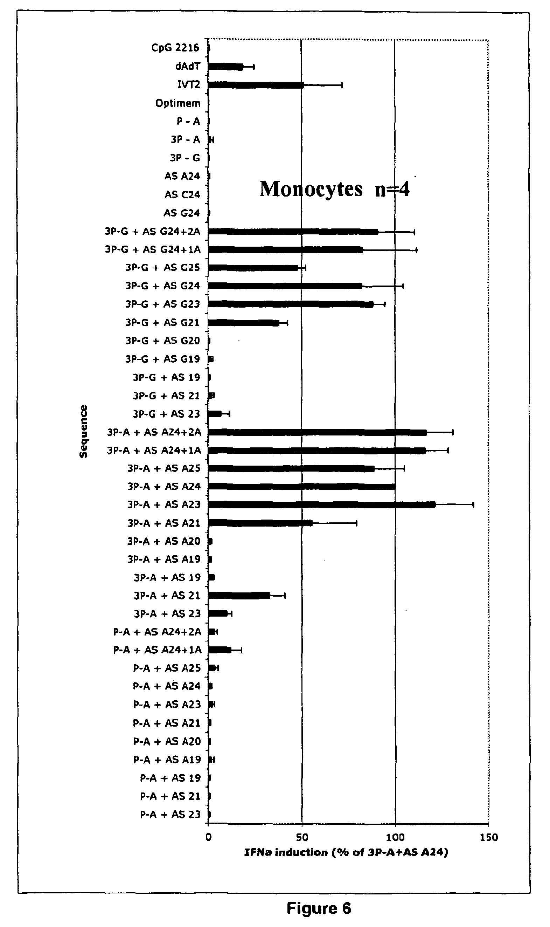

The present inventors stimulated purified primary human monocytes with the same synthetic blunt-ended short dsRNA without any 5' phosphate as that used in Marques TJ et al. (2006) ¹⁶, and found surprisingly that there was no IFN-α production (Example 2; Figure 2, sample "27+0 ds"). Furthermore, the present inventors stimulated purified primary human monocytes with a synthetic blunt-ended short dsRNA bearing a 5' phosphate similar to that used in Takahasi et al. (2008) ²⁴, and found surprisingly that there was little or no IFN-α production (Example 3; Figure 6, samples with "P-A" in the name). These findings contradict suggestions in the prior art that blunt-end was a molecular signature recognized by RIG-I and was capable of activating RIG-I in the absence of 5' triphosphate ^{16, 24}.

However, very surprisingly, when the present inventors stimulated purified primary human monocytes with synthetic dsRNA oligonucleotides bearing a 5' triphosphate, they found that the IFN-inducing activity of the dsRNA oligonucleotides was dramatically enhanced when the end bearing the 5' triphosphate was blunt (Example 3; Figures 4-6, samples "3P-X + AS X24", "3P-X + AS X24+A", "3P-X + AS X24+2A", "3P-X + AS X23"). The same results were obtained with peripheral blood mononuclear cells (PBMC) depleted of PDCs or PBMCs pre-treated with chloroquine, in which cases IFN-α production from PDCs was excluded.

This finding is surprising because it contradicts earlier report that the presence of blunt end did not enhance the IFN-inducing activity of dsRNA bearing 5' triphosphate ¹⁵. Furthermore, this finding demonstrates for the first time that the blunt end has to be on the same side as the 5' triphosphate to be recognized by and activate RIG-I.

This surprising finding suggests that both 5' triphosphate and blunt end are molecular signatures recognized by RIG-I. Whereas 5' triphosphate is the primary molecular signature recognized by RIG-I, blunt end is a secondary one which is not capable of activating RIG-I on its own but is capable of augmenting RIG-I activation in the presence of 5' triphosphate. Furthermore, the fact that the activity-enhancing effect of the blunt end was only observed when the blunt end was the end that bore the 5' triphosphate suggests that 5' triphosphate and blunt end are recognized by functional domains or sub-domains of RIG-I which are adjacent to each other in the 3-dimensional structure. In particular, it is highly likely that 5' triphosphate and blunt end are recognized by the same domain of RIG-I, the C-terminal regulatory domain.

The present finding is surprising also because the present inventors found that dsRNA oligonucleotides as short as 21 base pairs (bp) were capable of activating RIG-I and inducing significant IFN-α production when they bore 5' triphosphate and a blunt end, which is in contrast to the suggestion of Marques et al. (2006) that a length of 25bp was required for consistent RIG-I activation ¹⁶.

Moreover, the present inventors found that the RIG-I-activating and IFN-α-inducing activity of a dsRNA bearing 5' triphosphate and blunt end depended on the identity of the 5' nucleotide bearing the 5' triphosphate. Whereas a dsRNA having a 5' adenosine (A) was more potent than one having a 5' guanosine (G) or one having a 5' uridine (U), they were all more potent than one having a 5' cytidine (C).

It is hypothesized that chemically synthesized dsRNA oligonucleotides are essentially homogenous populations, wherein the oligonucleotides in each population are chemically well-defined and have essentially the same length, sequence and end structures. In contrast, dsRNA oligonucleotides obtained via *in vitro* transcription have variable lengths and end structures.

The present inventors have thus identified synthetic dsRNA oligonucleotides bearing at least one 5' triphosphate and at least one blunt end at the same end as the 5' triphosphate, with a reference for A as the 5' triphoshpate-bearing nucleoside, as highly potent agents for activating RIG-I and inducing type I IFN production from RIG-I-expressing cells.

### Definitions

As used herein, "a" and "an" refer to not only a single individual, but also a group or species of entities unless otherwise noted.

All terms used herein bear the meanings that are established in the art unless otherwise noted. Techniques disclosed herein can be performed by a person skilled in the art following the present description and/or established protocols, such as those disclosed in Molecular Cloning: A Laboratory Manual (Sambrook et al., 1989, Cold Spring Harbour Laboratory, New York), Current Protocols in Molecular Biology (Ausubel et al., 2007, John Wiley & Sons, New York), and Current Protocols in Immunology (Coligan et al., 2007, John Wiley & Sons, New York).

The expressions "an RNA oligonucleotide bearing 5' triphosphate", "triphosphate RNA oligonucleotide" and "3pRNA oligonucleotide" are used interchangeably.

The expressions "structural motif' and "molecular signature" are used interchangeably.

### Oligonucleotides

The present invention provides an oligonucleotide preparation which comprises an essentially homogenous population of an oligonucleotide and which is capable of activating RIG-I and inducing an anti-viral response, in particular, a type I IFN response, more specifically, an IFN-α response, as further defined in the claims.

The oligonucleotide has two blunt ends and comprises at least 1 ribonucleotide at the 5' end at the blunt end, wherein the blunt end bears a 5' triphosphate attached to the 5' ribonucleotide, wherein the 5' triphosphate is free of any cap structure, and wherein the blunt ends are ends of a fully double-stranded section which is at least 21 base pair (bp) in length and at most 30 bp in length, as defined in the claims. In other words, the blunt ends are the ends of the fully double-stranded section.

By "fully double-stranded", it is meant that the double-stranded section is not interrupted by any single-stranded structures. An oligonucleotide section is fully double-stranded when the two strands forming the section have the same length and the sequences of the two strands are 100% complementary to each other. As established in the art, two nucleotides are said to be complementary to each other if they can form a base pair, either a Waston-Crick base pair (A-U, G-C) or a wobble base pair (U-G, U-A, I-A, I-U, I-C).

The double-stranded section is preferably at least 22, more preferably 23, even more preferably at least 24 bp in length. The double-stranded section is at most 30 bp in length.

The oligonucleotide has preferably at least 2, 3, 4, 5, 6, more preferably at least 7, 8, 9, 10, 11, 12, even more preferably, at least 13, 14, 15, 16, 17, 18, most preferably, at least 19, 20, 21 ribonucleotides at the 5' end of the strand bearing the 5' triphosphate. In the most preferred embodiment, the fully double-stranded section is composed solely of ribonucleotides.

In one embodiment, the oligonucleotide is an RNA oligonucleotide. In another embodiment, the oligonucleotide is an RNA-DNA hybrid.

By "an essentially homogenous population", it is meant that the oligonucleotides contained in the preparation have essentially the same chemical identity (or chemical composition), including the same nucleotide sequence, backbone, modifications, length, and end structures. In other words, the oligonucleotides contained in the preparation are chemically defined and are essentially identical to each other. In particular, it is meant that at least 85%, preferably at least 90%, more preferably at least 95%, even more preferably at least 96%, 97%, 98%, most preferably at least 99% of the oligonucleotides in the preparation have the same chemical identity (or chemical composition), including the same nucleotide sequence, backbone, modifications, length, and end structures. In other words, the preparation is at least 85%, preferably at least 90%, more preferably at least 95%, even more preferably at least 96%, 97%, 98%, most preferably at least 99% pure. The purity and the chemical identity (or chemical composition) of an oligonucleotide preparation can be readily determined by a person skilled in the art using any appropriate methods, such as gel electrophoresis (in particular, denaturing gel electrophoresis), HPLC, mass spectrometry (e.g., MALDI-ToF MS), and sequencing.

The oligonucleotide is a double-stranded oligonucleotide.

Specifically, the double-stranded oligonucleotide is one wherein one of the strands comprises at least 1, preferably at least 3, more preferably at least 6 ribonucleotide(s) at the 5' end, and has a triphosphate attached to the most 5' ribonucleotide, wherein the triphosphate is free of any cap structure and wherein the ends are blunt ends, as defined in the claims .

The double-stranded oligonucleotide has one blunt end bearing the 5' triphosphate and a second blunt end which does not bear a 5' triphosphate.

The double-stranded oligonucleotide has one 5' triphosphate. The double-stranded oligonucleotide is at least 21bp in length, wherein the length refers to the number of base pairs of the continuous section of the oligonucleotide that is fully double-stranded. By "continuous", it is meant that the section of the oligonucleotide that is completely double-stranded and is not interrupted by any single-stranded structures. Preferably, the double-stranded oligonucleotide is at least 22, more preferably at least 23, and most preferably at least 24 bp in length. Preferably, the double-stranded oligonucleotide is at most 30bp in length.

In one embodiment, the double-stranded oligonucleotide is a homoduplex. By "homoduplex", it is meant that the two strands forming the oligonucleotide have exactly the same length and sequence in the 5' to 3' orientation. A homoduplex can be formed when each strand forming the double-stranded oligonucleotide has a sequence that is 100% self-complementary, meaning that the sequence of the 5' half is 100% complementary to that of the 3' half.

Various methods for producing oligonucleotides are known in the art. However, in order to obtain an essentially homogeneous population of a double-stranded oligonucleotide, as defined in the claims, chemical synthesis is the method of preparation. The particular method or process of chemical synthesis is not important; it is only important that the synthesized oligonucleotide is purified and quality-controlled such that the oligonucleotide preparation contains essentially a homogenous population of oligonucleotides having essentially the same chemical identity (or chemical composition), including the same nucleotide sequence, backbone, modifications, length, and end structures, as defined in the claims. The oligonucleotides can be purified by any standard methods in the art, such as capillary gel electrophoresis and HPLC. Synthetic oligonucleotides, double-stranded, obtained from most commercial sources contain 5' OH. These synthetic oligonucleotides can be modified at the 5' end to bear a 5' triphosphate by any appropriate methods known in the art. The preferred method for 5' triphosphate attachment is that developed by János Ludwig and Fritz Eckstein ²⁷.

Alternatively, *in vitro* transcription can be employed, which is disclosed but not claimed. However, in order to obtain the single strands to prepare the a double-stranded oligonucleotide by in vitro transcription, measures need to be taken to ensure that each intended in vitro transcribed single strand is indeed single-stranded. Aberrant transcripts may be generated in vitro using an RNA polymerase. For example, it is hypothesized that an RNA transcript generated by an RNA polymerase in vitro may fold back onto itself and prime RNA-dependent RNA synthesis, leading to the generation of aberrant transcripts of undefined and/or nonuniform lengths and sequences. Therefore, in principle, any measure that would prevent RNA synthesis primed by the RNA transcript itself can be employed.

For example, a single stranded oligonucleotide is designed to have a sequence X₁-X₂-X₃-...X_{m- 2}-Xₘ₋₁-Xₘ, wherein m is the length of the oligonucleotide, wherein the sequence has no or minimal self complementarity, wherein X₁, X₂, X₃, ..., Xₘ are chosen from 1, 2 or 3 of the 4 conventional nucleotides A, U, C and G, wherein at least one of the nucleotides that are complementary to any of Xₘ₋₂, Xₘ₋₁, and Xₘ, i.e., Yₘ₋₂, Yₘ₋₁, and Yₘ, is not among the 1, 2, or 3 nucleotides chosen for X₁, X₂, X₃, ..., Xₘ.

An appropriate DNA template for generating such an ssRNA oligonucleotide can be generated using any appropriate methods known in the art. An in vitro transcription reaction is set up using the DNA template and a nucleotide mixture which does not contain the complementary nucleotide(s) which is(are) not comprised in X₁-X₂-X₃-...Xₘ₋₂-Xₘ₋₁-Xₘ. Any appropriate in vitro transcription conditions known in the art can be used. Due to the absence of the complementary nucleotide, no aberrant RNA-primed RNA synthesis can take place. As a result, a single-stranded population of X₁-X₂-X₃-...-Xₘ can be obtained. The resulting ssRNA preparation can be purified by any appropriate methods known in the art and an equal amount of two purified ssRNA preparations with complementary sequence can be annealed to obtain an essentially homogenous population of a double-stranded RNA oligonucleotide of desired sequence.

For example, the ssRNA oligonucleotide may be GACACACACACACACACACACACA (SEQ ID NO: 44) and the in vitro transcription may be carried out in the presence of ATP, CTP and GTP, i.e., in the absence of UTP, which is disclosed but not claimed.

It is also possible to synthesize the two strands forming the double-stranded oligonucleotide using different methods, which is disclosed but not claimed. For example, one strand can be prepared by chemical synthesis and the other by in vitro transcription. Furthermore, if desired, an in vitro transcribed ssRNA can be treated with a phosphatase, such as calf intestine phosphotase (CIP), to remove the 5' triphosphate.

Mismatch of one or two nucleotides may be tolerated in the double-stranded section of the oligonucleotide in that the IFN-inducing activity of the oligonucleotide is not significantly reduced. The mismatch is preferably at least 6bp, more preferably at least 12bp, even more preferably at least 18pb away from the blunt end bearing the 5' triphosphate.

In one embodiment, the double-stranded RNA oligonucleotide, as defined in the claims, contains one or more GU wobble base pairs instead of GC or UA base pairing.

The double-stranded oligonucleotide as defined in the claims comprises at least 1, 2, 3, 4, 5, preferably at least 6, 7, 8, 9, 10, more preferably at least 11, 12, 13, 14, 15, even more preferably at least 16, 17, 18, 19, 20 inosine (I). In another preferred embodiment, at least 1, 2, 3, 4, 5%, preferably at least 10, 15, 20, 25, 30%, more preferably at least 35, 40, 45, 50, 55, 60%, even more preferably at least 70, 80, or 90% of the adenosine (A) and/or guanosine (G) in the oligonucleotide is replaced with inosine (I). The 5' ribonucleotide bearing the at least one 5' triphosphate is preferably G, followed by U, followed by C, in the order of descending preference.

It is disclosed but not claimed that the sequence of the first 4 ribonucleotides at the 5' end of the double- or single-stranded oligonucleotide bearing the 5' triphosphate is selected from: AAGU (No. 1), AAAG (No. 2), AUGG (No. 3), AUUA (No. 4), AACG (No. 5), AUGA (No. 6), AGUU (No. 7), AUUG (No. 8), AACA (No. 9), AGAA (No. 10), AGCA (No. 11), AACU (No. 12), AUGG (No. 13), AGGA (No. 14), AUCA (No. 15), AUGC (No. 16), AGUA (No. 17), AAGC (No. 18), AACC (No. 19), AGGU (No. 20), AAAC (No. 21), AUGU (No. 22), ACUG (No. 23), ACGA (No. 24), ACAG (No. 25), AAGG (No. 26), ACAU (No. 27), ACGC (No. 28), AAAU (No. 29), ACGG (No. 30), AUUC (No. 31), AGUG (No. 32), ACAA (No. 33), AUCC (No. 34), AGUC (No. 35), wherein the sequence is in the 5'->3' direction. It is also disclosed that the sequence of the first 3 ribonucleotides at the 5' end of the oligonucleotide bearing the 5' triphosphate is selected from Nos. 1-19, more preferably from Nos. 1-9, even more preferably from Nos. 1-4. According to the invention, the first nucleotide of the above-listed 5' 4-nucleotide sequences is a G, U or C, in the order of descending preference, instead of A.

In certain embodiments, the double-stranded oligonucleotide contains one or more structural motif(s) or molecular signature(s) which is(are) recognized by the TLRs, in particular, TLR3, TLR7 and TLR8.

In one embodiment, the double-stranded oligonucleotide is at least 30bp in length and is recognized by TLR3 ².

In another embodiment, the double-stranded oligonucleotide contains defined sequence motifs recognized by TLR7 ^{3, 4, 5, 22, 29}. The double-stranded oligonucleotide can comprise at least one, preferably at least two, more preferably at least three, even more preferably at least four, even more preferably at least five, and most preferably at least six, of the 4-nucleotide (4mer) motifs selected from the group consisting of:
GUUC (No. 101), GUCA (No. 102), GCUC (No. 103), GUUG (No. 104), GUUU (No. 105), GGUU (No. 106), GUGU (No. 107), GGUC (No. 108), GUCU (No. 109), GUCC (No. 110), GCUU (No. 111), UUGU (No. 112), UGUC (No. 113), CUGU (No. 114), CGUC (No. 115), UGUU (No. 116), GUUA (No. 117), UGUA (No. 118), UUUC (No. 119), UGUG (No. 120), GGUA (No. 121), GUCG (No. 122), UUUG (No. 123), UGGU (No. 124), GUGG (No. 125), GUGC (No. 126), GUAC (No. 127), GUAU (No. 128), UAGU (No. 129), GUAG (No. 130), UUCA (No. 131), UUGG (No. 132), UCUC (No. 133), CAGU (No. 134), UUCG (No. 135), CUUC (No. 136), GAGU (No. 137), GGUG (No. 138), UUGC (No. 139), UUUU (No. 140), CUCA (No. 141), UCGU (No. 142), UUCU (No. 143), UGGC (No. 144), CGUU (No. 145), CUUG (No. 146), UUAC (No. 147),
wherein the nucleotide sequences of the motifs are 5' → 3'.

Preferably, the 4mer motifs are selected from the group consisting of Nos. 101-119, Nos. 101-118, Nos. 101-117, Nos. 101-116, more preferably, Nos. 101-115, Nos. 101-114, Nos. 101-113, Nos. 101-112, more preferably, Nos. 101-111, Nos. 110-110, Nos. 101-109, No. 101-108, Nos. 101-107, even more preferably, Nos. 101-106, Nos. 101-105, Nos. 101-104, Nos. 101-103, most preferably, Nos. 101-102 of the 4mer motifs. The oligonucleotide may comprise one or more copies of the same 4mer motif or one or more copies of one or more different 4mer motifs.

The double-stranded oligonucleotide may contain one or more of the same or different structural motif(s) or molecular signature(s) recognized by TLR3, TLR7, TLR8 and TLR9 as described above.

In certain embodiments, the double-stranded oligonucleotide as defined in the claims has gene-silencing activity. As used herein, the term "gene-silencing activity" refers to the capability of the oligonucleotide to downregulate the expression of a gene, preferably via RNA interference (RNAi). In a preferred embodiment, the oligonucleotide is an siRNA (small interfering RNA) or an shRNA (small hairpin RNA).

Given the coding sequence of a gene, a person skilled in the art can readily design siRNAs and shRNAs using publicly available algorithms such as that disclosed in Reynolds et al. ²³ and design engines such as "BD-RNAi design" (Beckton Dickinson) and "Block-iT RNAi" (Invitrogen). Even though conventional siRNAs usually are 19bp in length and have two 2-nucleotide 3' overhangs (i.e., each single strand is 21 nucleotides in length), a person skilled in the art can readily modify the sequence of the siRNAs designed by the known algorithms or design engines and obtain double-stranded oligonucleotides which have the structural characteristics of those defined in the claims . Moreover, a person skilled in the art can readily test the gene-silencing efficacy of the oligonucleotides using methods known in the art such as Northern blot analysis, quantitative or semi-quantitative RT-PCR, Western blot analysis, surface or intracellular FACS analysis.

In a preferred embodiment, the siRNA is at least 21bp in length; the sense strand bears a 5' triphosphate; the end that bears the 5' triphosphate is a blunt end and the other end is a blunt end, as defined in the claims .

In preferred embodiments, the siRNA or shRNA is specific for a disease/disorder-associated gene. As widely used in the art, the term " a disease/disorder-related gene" refers to a gene that is expressed in a cell in a disease/disorder but not expressed in a normal cell or a gene that is expressed at a higher level in a cell in a disease/disorder than in a normal cell. In a preferred embodiment, the expression of the disease/disorder-associated gene causes or contributes to the establishment and/or progression of the disease/disorder.

In one embodiment, the disease/disorder is a tumor or a cancer and the disease/disorder-associated gene is an oncogene. Examples of oncogenes include wild-type and/or mutant Bcl-2, c-Myc, c-Ras, c-Met, Her2, EGFR, PDGFR, VEGFR, Edg4, Edg7, S1P, Raf, ERKWNT, Survivin, HGF, cdk2, cdk4, MITF, cyclin D1, GRO.

In another embodiment, the disease/disorder is a viral infection and the disease/disorder-associated gene is a gene which is or the product of which is required for host cell recognition, host cell entry, viral replication, viral partical assembly, and/or viral transmission. The disease/disorder-associated gene may be a viral gene or a host gene. An example of a viral gene is HbsAg of HBV.

The double-stranded oligonucleotide may contain any naturally-occurring, synthetic, modified nucleotides, or a mixture thereof, as long as the synthetic and/or modified nucleotides do not compromise (i.e., reduce) the type I IFN-inducing activity of the oligonucleotide. The oligonucleotide does not contain any modification such as pseudouridine, 2-thiouridine, 2'-Fluorine-dNTP, 2'-O-methylated NTP, in particular 2'-fluorine-dCTP, 2'-fluorine-dUTP, 2'-O-methylated CTP, 2'-O-methylated UTP. The double-stranded oligonucleotide may contain any naturally-occurring, synthetic, modified internucleoside linkages, or a mixture thereof, as long as the linkages do not compromise the type I IFN-inducing activity of the oligonucleotide. The 5' triphosphate group of the double-stranded oligonucleotide may be modified as long as the modification does not compromise the type I IFN-inducing activity of the oligonucleotide. For example, one or more of the oxygen (O) in the triphosphate group may be replaced with a sulfur (S); the triphosphate group may be modified with the addition of one or more phosphate group(s).

The double-stranded oligonucleotide may be modified covalently or non-covalently to improve its chemical stability, resistance to nuclease degradation, ability to across cellular and/or subcellular membranes, target (organ, tissue, cell type, subcellular compartment)-specificity, pharmacokinetic properties, biodistribution, or any combinations thereof. For example, phosphorothioate linkage(s) and/or pyrophosphate linkage(s) may be introduced to enhance the chemical stability and/or the nuclease resistance of an RNA oligonucleotide. In another example, the oligonucleotide may be covalently linked to one or more lipophilic group(s) or molecule(s), such as a lipid or a lipid-based molecule, preferably, a cholesterol or a derivative thereof. The lipophilic group or molecule is preferably not attached to the blunt end bearing the 5' triphosphate. Preferably, the modification does not comprise the type I IFN-inducing activity of the oligonucleotide. Alternatively, a reduction in the type I IFN-inducing activity of the oligonucleotide caused by the modification is off-set by an increase in the stability and/or delivery and/or other properties as described above.

The double-stranded oligonucleotide may bear any combination of any number of features described above, as further defined in the claims. A preferred double-stranded oligonucleotide is an RNA oligonucleotide having one blunt end with one 5' triphosphate attached to a 5' and a length of between 21 and 30bp, as further defined I the claims. A more preferred double-stranded oligonucleotide is an RNA oligonucleotide having two blunt ends bearing one 5' triphosphate attached to a 5' and a length of between 21 and 30bp.

### Pharmaceutical Composition

The present invention provides a pharmaceutical composition comprising at least one of the oligonucleotide preparation of the invention described above and a pharmaceutically acceptable carrier, as defined in the claims.

By "at least one", it is meant that one or more oligonucleotide preparation(s) of the same or different oligonucleotide(s) can be used together.

In a preferred embodiment, the pharmaceutical composition further comprises an agent which facilitates the delivery of the oligonucleotide into a cell, in particular, into the cytosol of the cell, as defined in the claims.

In one embodiment, the delivery agent is a complexation agent which forms a complex with the oligonucleotide and facilitates the delivery of the oligonucleotide into cells. Complexation agents are also referred to as "transfection agents" in the art. Any complexation agent which is compatible with the intended use of the pharmaceutical composition can be employed. Examples of complexation agents include polymers and biodegradable microspheres. The polymer is preferably a cationic polymer, more preferably a cationic lipid. Examples of a polymer include polyethylenimine (PEI) such as in vivo-jetPEI^{™} (PolyPlus) and collagen derivatives. Examples of biodegradable microspheres include liposomes, virosomes, stable-nucleic-acid-lipid particles (SNALPs), SICOMATRIX^{®} (CSL Limited), and poly (D,L-lactide-co-glycolide) copolymer (PLGA) microspheres.

In another embodiment, the delivery agent is a virus, preferably a replication-deficient virus. The oligonucleotide to be delivered is contained in the viral capsule and the virus may be selected based on its target specificity. Examples of useful viruses include polymyxoviruses which target upper respiratory tract epithelia and other cells, hepatitis B virus which targets liver cells, influenza virus which targets epithelial cells and other cells, adenoviruses which targets a number of different cell types, papilloma viruses which targets epithelial and squamous cells, herpes virus which targets neurons, retroviruses such as HIV which targets CD4⁺ T cells, dendritic cells and other cells, modified Vaccinia Ankara which targets a variety of cells, and oncolytic viruses which target tumor cells. Examples of oncolytic viruses include naturally occurring wild-type Newcastle disease virus, attenuated strains of reovirus, vesicular stomatitis virus (VSV), and genetically engineered mutants of herpes simplex virus type 1 (HSV-1), adenovirus, poxvirus and measles virus.

In addition to being delivered by a delivery agent, the oligonucleotide and/or the pharmaceutical composition can be delivered into cells via physical means such as electroporation, shock wave administration, ultrasound triggered transfection, and gene gun delivery with gold particles.

The pharmaceutical composition may further comprise another agent such as an agent that stabilizes the oligonucleotide. Examples of a stabilizing agent include a protein that complexes with the oligonucleotide to form an iRNP, chelators such as EDTA, salts, and RNase inhibitors.

In certain embodiments, the pharmaceutical composition further comprises one or more pharmaceutically active therapeutic agent(s), as defined in the claims. Examples of a pharmaceutically active agent include immunostimulatory agents, anti-viral agents, antibiotics, anti-fungal agents, anti-parasitic agents, anti-tumor agents, cytokines, chemokines, growth factors, anti-angiogenic factors, chemotherapeutic agents, antibodies and gene silencing agents. Preferably, the pharmaceutically active agent is selected from the group consisting of an immunostimulatory agent, an anti-viral agent and an anti-tumor agent. The more than one pharmaceutically active agents may be of the same or different category.

In certain embodiments, the pharmaceutical composition further compriese retinoid acid, IFN-α and/or IFN-β. Retinoid acid, IFN-α and/or IFN-β are capable of sensitizing cells for IFN-α production, possibly through the upregulation of RIG-I expression.

The pharmaceutical composition may be formulated in any way that is compatible with its therapeutic application, including intended route of administration, delivery format and desired dosage. Optimal pharmaceutical compositions may be formulated by a skilled person according to common general knowledge in the art, such as that described in Remington's Pharmaceutical Sciences (18th Ed., Gennaro AR ed., Mack Publishing Company, 1990).

The pharmaceutical composition may be formulated for instant release, controlled release, timed-release, sustained release, extended release, or continuous release.

The pharmaceutical composition may be administered by any route known in the art, including, but not limited to, topical, enteral and parenteral routes, provided that it is compatible with the intended application. Topic administration includes, but is not limited to, epicutaneous, inhalational, intranasal, vaginal administration, enema, eye drops, and ear drops. Enteral administration includes, but is not limited to, oral, rectal administration and administration through feeding tubes. Parenteral administration includes, but is not limited to, intravenous, intraarterial, intramuscular, intracardiac, subcutaneous, intraosseous, intradermal, intrathecal, intraperitoneal, transdermal, transmucosal, and inhalational administration.

The pharmaceutical composition is for local (e.g., mucosa, skin) applications, such as in the form of a spray (i.e., aerosol) preparation.

The pharmaceutical composition may be use for prophylactic and/or therapeutic purposes. For example, a spray (i.e., aerosol) preparation may be used to strengthen the anti-viral capability of the nasal and the pulmonary mucosa.

The optimal dosage, frequency, timing and route of administration can be readily determined by a person skilled in the art on the basis of factors such as the disease or condition to be treated, the severity of the disease or condition, the age, gender and physical status of the patient, and the presence or absence of previous treatment.

### In Vitro Applications

The present application provides the in vitro use of the oligonucleotide preparation of the invention described above and further defined in the claims. In particular, the present application provides the use of at least one oligonucleotide preparation for inducing an anti-viral response, in particular, a type I IFN response, more specifically, an IFN-α response, *in vitro,* as defined in the claims. The present application also provides the use of at least one oligonucleotide preparation for inducing apoptosis of a tumor cell in vitro.

The present invention provides an *in vitro* method for stimulating an anti-viral response, in particular, a type I IFN response, more specifically, an IFN-α response in a cell, as defined in the claims, comprising the steps of:
(a) mixing at least one oligonucleotide of the invention as defined in the claims with a complexation agent; and
(b) contacting a cell with the mixture of (a), wherein the cell expresses RIG-I.

The cells may express RIG-I endogenously and/or exogenously from an exogenous nucleic acid (RNA or DNA). The exogenous DNA may be a plasmid DNA, a viral vector, or a portion thereof. The exogenous DNA may be ingerated into the genome of the cell or may exisit extra-chromosomally. The cells include, but are not limited to, primary immune cells, primary non-immune cells, and cell lines. Immune cells include, but are not limited to, peripheral blood mononuclear cells (PBMC), plasmacytoid dendritric cells (PDC), myeloid dendritic cells (MDC), macrophages, monocytes, B cells, natural killer cells, granulocytes, CD4+ T cells, CD8+ T cells, and NKT cells. Non-immune cells include, but are not limited to, fibroblasts, endothelial cells, epithelial cells, and tumor cells. Cell lines may be derived from immune cells or non-immune cells.

The present disclosure provides an in vitro method for inducing apoptosis of a tumor cell, comprising the steps of:
(a) mixing at least one oligonucleotide of the invention described above with a complexation agent; and
(b) contacting a tumor cell with the mixture of (a)
which is not claimed.

The tumor cell may be a primary tumor cell freshly isolated from a vertebrate animal having a tumor or a tumor cell line.

### In Vivo Applications

The present application provides the *in vivo* use of the oligonucleotide preparation of the invention as defined in the claims .

In particular, the present invention provides at least one oligonucleotide preparation for inducing an anti-viral response, in particular, a type I IFN response, more specifically, an IFN-α response, in a vertebrate animal, in particular, a mammal, as further defined in the claims. The present application further provides at least one oligonucleotide preparation for inducing apoptosis of a tumor cell in a vertebrate animal, in particular, a mammal. The present application additionally provides at least one oligonucleotide preparation for preventing and/or treating a disease and/or disorder in a vertebrate animal, in particular, a mammal, in medical and/or veterinary practice, as further defined in the claims At least one oligonucleotide preparation can be used as a vaccine adjuvant. Furthermore, the present application provides the use of at least one oligonucleotide preparation for the preparation of a pharmaceutical composition for inducing an anti-viral response, in particular, a type I IFN response, more specifically, an IFN-α response, in a vertebrate animal, in particular, a mammal, as defined in the claims. The present application further provides the use of at least one oligonucleotide preparation for the preparation of a pharmaceutical composition for inducing apoptosis of a tumor cell in a vertebrate animal, in particular, a mammal. The present application additionally provides the use of at least one oligonucleotide preparation for the preparation of a pharmaceutical composition for preventing and/or treating a disease and/or disorder in a vertebrate animal, in particular, a mammal, in medical and/or veterinary practice, as defined in the claims.

The diseases and/or disorders include infections, tumors/cancers, and immune disorders, as defined in the claims.

Infections include, but are not limited to, viral infections, bacterial infections, anthrax, parasitic infections, fungal infections and prion infection.

Viral infections include, but are not limited to, infections by hepatitis C, hepatitis B, influenza virus, herpes simplex virus (HSV), human immunodeficiency virus (HIV), respiratory syncytial virus (RSV), vesicular stomatitis virus(VSV), cytomegalovirus (CMV), poliovirus, encephalomyocarditis virus (EMCV), human papillomavirus (HPV) and smallpox virus. In one embodiment, the infection is an upper respiratory tract infection caused by viruses and/or bacteria, in particular, flu, more specifically, bird flu.

Bacterial infections include, but are not limited to, infections by *streptococci*, *staphylococci*, *E. Coli*, and *pseudomonas.* In one embodiment, the bacterial infection is an intracellular bacterial infection which is an infection by an intracellular bacterium such as mycobacteria (tuberculosis), chlamydia, mycoplasma, listeria, and an facultative intracelluar bacterium such as staphylococcus aureus.

Parasitic infections include, but are not limited to, worm infections, in particular, intestinal worm infection.

In a preferred embodiment, the infection is a viral infection or an intracellular bacterial infection. In a more preferred embodiment, the infection is a viral infection by hepatitis C, hepatitis B, influenza virus, RSV, HPV, HSV1, HSV2, and CMV.

Tumors include both benign and malignant tumors (i.e., cancer).

Cancers include, but are not limited to biliary tract cancer, brain cancer, breast cancer, cervical cancer, choriocarcinoma, colon cancer, endometrial cancer, esophageal cancer, gastric cancer, intraepithelial neoplasm, leukemia, lymphoma, liver cancer, lung cancer, melanoma, myelomas, neuroblastoma, oral cancer, ovarian cancer, pancreatic cancer, prostate cancer, rectal cancer, sarcoma, skin cancer, testicular cancer, thyroid cancer and renal cancer.

In certain embodiments, the cancer is selected from hairy cell leukemia, chronic myelogenous leukemia, cutaneous T-cell leukemia, chronic myeloid leukemia, non-Hodgkin's lymphoma, multiple myeloma, follicular lymphoma, malignant melanoma, squamous cell carcinoma, renal cell carcinoma, prostate carcinoma, bladder cell carcinoma, breast carcinoma, ovarian carcinoma, non-small cell lung cancer, small cell lung cancer, hepatocellular carcinoma, basaliom, colon carcinoma, cervical dysplasia, and Kaposi's sarcoma (AIDS-related and non-AIDS related).

Immune disorders include, but are not limited to, allergies, autoimmune disorders, and immunodeficiencies.

Allergies inlclude, but are not limited to, respiratory allergies, contact allergies and food allergies.

Autoimmune diseases include, but are not limited to, multiple sclerosis, diabetes mellitus, arthritis (including rheumatoid arthritis, juvenile rheumatoid arthritis, osteoarthritis, psoriatic arthritis), encephalomyelitis, myasthenia gravis, systemic lupus erythematosis, autoimmune thyroiditis, dermatisis (including atopic dermatitis and eczematous dermatitis), psoriasis, Siogren's Syndrome, Crohn's disease, aphthous ulcer, iritis, conjunctivitis, keratoconjunctivitis, ulcerative colitis, asthma, allergic asthma, cutaneous lupus erythematosus, scleroderma, vaginitis, proctitis, drug eruptions, leprosy reversal reactions, erythema nodosum leprosum, autoimmune uveitis, allergic encephalomyelitis, acute necrotizing hemorrhagic encephalopathy, idiopathic bilateral progressive sensorineural hearing loss, aplastic anemia, pure red cell anemia, idiopathic thrombocytopenia, polychondritis, Wegener's granulomatosis, chronic active hepatitis, Stevens-Johnson syndrome, idiopathic sprue, lichen planus, Graves' disease, sarcoidosis, primary biliary cirrhosis, uveitis posterior, and interstitial lung fibrosis.

Immunodeficiencies include, but are not limited to, spontaneous immunodeficiency, acquired immunodeficiency (including AIDS), drug-induced immunodeficiency or immunosuppression (such as that induced by immunosuppressants used in transplantation and chemotherapeutic agents used for treating cancer), and immunosuppression caused by chronic hemodialysis, trauma or surgical procedures.

In a preferred embodiment, the immune disorder is multiple sclerosis.

In certain preferred embodiments, the oligonucleotide has gene silencing activity. The oligonucleotide thus has two functionalities, gene silencing and immune stimulating, combined in one molecule. In preferred embodiments, the oligonucleotide has gene-silencing activity that is specific for a disease/disorder-associated gene, such as an oncogene or a gene required for viral infection and/or replication.

In certain embodiments, the oligonucleotide is used in combination with one or more pharmaceutically active agents such as immunostimulatory agents, anti-viral agents, antibiotics, anti-fungal agents, anti-parasitic agents, anti-tumor agents, cytokines, chemokines, growth factors, anti-angiogenic factors, chemotherapeutic agents, antibodies and gene silencing agents. Preferably, the pharmaceutically active agent is selected from the group consisting of an immunostimulatory agent, an anti-viral agent and an anti-tumor agent. The more than one pharmaceutically active agents may be of the same or different category.

In one embodiments, the oligonucleotide is used in combination with an anti-viral vaccine, an anti-bacterial vaccine, and/or an anti-tumor vaccine, wherein the vaccine can be prophylactic and/or therapeutic. The oligonucleotide can serve as a vaccine adjuvant.

In another embodiment, the oligonucleotide is used in combination with retinoic acid and/or type I IFN (IFN-α and/or IFN-β). Retinoid acid, IFN-α and/or IFN-β are capable of sensitizing cells for IFN-α production, possibly through the upregulation of RIG-I expression.

In other embodiments, the pharmaceutical composition is for use in combination with one or more prophylactic and/or therapeutic treatments of diseases and/or disorders such as infection, tumor, and immune disorders. The treatments may be pharmacological and/or physical (e.g., surgery, radiation).

Vertebrate animals include, but are not limited to, fish, amphibians, birds, and mammals. Mammals include, but are not limited to, rats, mice, cats, dogs, horses, sheep, cattle, cows, pigs, rabbits, non-human primates, and humans. In a preferred embodiment, the mammal is human.

The present invention is illustrated by the following Examples. The Examples are for illustration purposes only and are by no means to be construed to limit the scope of the invention.

### EXAMPLES

### Materials and Methods

### 1. Cells

Human PBMC were prepared from whole blood donated by young healthy donors by Ficoll-Hypaque density gradient centrifugation (Biochrom, Berlin, Germany). PDC were isolated by MACS using the blood dendritic cell Ag (BCDA)-4 dendritic cell isolation kit from Miltenyi Biotec (Bergisch-Gladbach, Germany). Briefly, PDC were labelled with anti-BDCA-4 Ab coupled to colloidal paramagnetic microbeads and passed through a magnetic separation column twice (LS column, then MS column; Miltenyi Biotec). The purity of isolated PDC (lineage-negative, MHC-II-positive and CD123-positive cells) was above 95 %. Before isolation of monocytes, PDC were depleted by MACS (LD column; Miltenyi Biotec) and then monocytes were isolated using the monocyte isolation kit II (Miltenyi Biotec). MDCs were purified from PBMC by immunomagnetic sorting with anti-CD1c beads (CD1c (BDCA-1)+ Dendritic Cell Isolation Kit, human, Miltenyi Biotec). Viability of all cells was above 95 %, as determined by trypan blue exclusion. Unless indicated otherwise, cells were cultured in 96-well plates for stimulation experiments. MDCs (0.5×10⁶/ml) were kept in RPMI 1640 containing 10% FCS, 1.5mM L-glutamine, 100 U/ml penicilin and 100 µg/ml streptomycin. PDCs (0.25×10⁶/ml) were cultured in the same medium supplemented with 10 ng/ml IL-3 (R&D Systems GmbH). Monocytes (0.5×10⁶/ml) were resuspended in RPMI medium with 2% AB serum (BioWhittaker, Heidelberg, Germany), 1.5mM L-glutamine, 100 U/ml penicilin and 100 µg/ml streptomycin. All compounds were tested for endotoxin contamination prior to use.

### 2. Mice

TLR7-deficient (TLR7^{-/-}) mice were kindly provided by S. Akira ³. Female C57BU6 mice were purchased from Harlan-Winkelmann (Borchen, Germany). Mice were 6-12 weeks of age at the onset of experiments. Animal studies were approved by the local regulatory agency (Regierung von Oberbayern, Munich, Germany).

### 3. RNAs

Chemically synthesized RNA oligonucleotides were purchased from Eurogentec (Leiden, Belgium), MWG-BIOTECH AG (Ebersberg, Germany), biomers.net GmbH (Ulm, Germany), and optionally modified by Janos Ludwig (Rockefeller Univ., USA). *In vitro* transcribed RNAs were prepared using the Megashort Script Kit (Ambion, Huntingdon, UK) following the manufacture's instructions. *In vitro* transcription was carried out overnight at 37 °C. The DNA template was digested using DNase I (Ambion). RNAs were purified by phenol:chloroform extraction and alcohol precipitation. Excess salts and NTPs were removed by passing the RNAs through a Mini Quick Spin^{™} Oligo Column (Roche). Size and integrity of RNAs was checked via gel electrophoresis. CpG-ODN was purchased from Coley Pharmaceutical Group (Wellesley, USA) or Metabion (Martinsried, Germany).

### 4. Cell Stimulation (transfection)

Unless otherwise indicated, 200ng of the purified RNA oligonucleotides were transfected into cells using 0.5µl of Lipofectamine 2000 (Invitrogen) in each well of a 96-well plate according to the manufacturer s protocol. CpG ODN was used at a final concentration of 3 µg/ml. For transfection with the polycationic polypeptide poly-L-arginine (Sigma, P7762), 200ng nucleic acid diluted in PBS (PAA Laboratories GmBH) were mixed with 280ng poly-L-arginine and incubated for 20min prior to stimulation. In some experiments, cells were pre-treated with 1 or 2.5 µg/ml chloroquine (Sigma) for 30min prior to stimulation. 24h post-stimulation/transcription, tissue culture supernatants were collected and assayed for IFN-α production.

### 5. IFN-α ELISA

Human IFN-α was assessed in cell culture supernatants harvested 24 hours after stimulation/transfection using the IFN-α module set (Bender MedSystems, Graz, Austria) according to manufacturer's recommendations. Murine IFN-α was measured according to the following protocol: monoclonal rat anti-mouse IFN-α (clone RMMA-1) was used as the capture Ab, polyclonal rabbit anti-mouse IFN-α serum was used for detection (both PBL Biomedical Laboratories), and HRP-conjugated donkey anti-rabbit IgG was used as the secondary reagent (Jackson ImmunoResearch Laboratories). Mouse rlFN-α (PBL Biomedical Laboratories) was used as the standard (IFN-α concentration in IU/ ml).

### Results

### Example 1. RNA oligonucleotides bearing 5' triphosphate are recognized by different receptors in monocytes and PDCs. (Comparative example)

PDCs, MDCs and monocytes were all found to be able to respond to stimulation of RNA oligonucleotides bearing 5' triphosphate (3pRNA) by producing IFN-α (data not shown). To identify the receptors involved in the recognition of 3pRNA in these different immune cell populations, in vitro transcribed 3pRNA (Table 1) were transfected into these cells in the presence and absence of chloroquine, a potent inhibitor of TLR7, TLR8 and TLR9-mediated nucleic aid recognition.

**Table 1. RNA and DNA oligonucleotide sequences.**

| name | sequence | type | SEQ ID NO |
|---|---|---|---|
| GA | 5'-pppGGGGGGGGGGGAAAAAAAAAAAA-3' | RNA, in vitro transcribed | 1 |
| GFPs | 5'-pppGGGGCUGACCCUGAAGUUCAUCUU-3' | RNA, in vitro transcribed | 2 |
| SynRNA | 5'-OHGGGGCUGACCCUGAAGUUCAUCUU-3' | RNA, synthetic | 2 |
| 3pRNA | 5'-pppGGGGCUGACCCUGAAGUUCAUCUU-3' | RNA, in vitro transcribed | 2 |
| CpG | 5'-GGGGGA**CG**AT**CG**T**C****G**GGGGG-3' | DNA, synthetic | 3 |

| | | | |
|---|---|---|---|
| (ppp: triphosphate; under lined letters: phosphorothioate linkage 3' of the base; bold letters, CpG dinucleotides) | | | |

As shown in Figure 1A, whereas 3pRNA-induced IFN-α production from monocytes was not affected by the addition of chloroquine, 3pRNA-induced IFN-α production from PDCs was greatly diminished by the addition of chloroquine.

Furthermore, as shown in Figure 1A, the ability of 3pRNA to induce IFN-α from PDCs depended on the presence of U in the sequence which is known to be a molecular signature recognized by TLR7.

Moreover, as shown in Figure 1B, whereas PDCs from wild-type mice responded to stimulation by 3pRNA by producing IFN-α, this response was dramatically diminished, if not completely absent, in PDCs from TLR7-deficient (TLR7^{-/-}) mice.

Taken together, these results suggest that whereas the recognition of 3pRNA in PDCs is primarily mediated by TLR7 in a nucleotide sequence-dependent manner, the recognition of 3pRNA in monocytes is primarily, if not entirely, mediated by RIG-I and is nucleotide sequence-independent.

### Example 2. IFN-α induction in monocytes strictly requires the presence of a 5' triphosphate.

Synthetic RNA oligonucleotides bearing 5' monophosphate (Table 2) were transfected into purified primary human monocytes. An in vitro transcribed RNA bearing 5' triphosphate was used as a positive control. The level of IFN-α secretion was determined 24 hours after transfection/stimulation.

**Table 2. RNA oligonucleotide sequences.**

| name | sequence | type | SEQ ID NO |
|---|---|---|---|
| 27+0 s | 5'-OHAAGCUGACCCUGAAGUUCAUCUGCACC-3' | RNA, synthetic | 4 |
| 27+0 a | 5'-OHGGUGCAGAUGAACUUCAGGGUCAGCUU-3' | RNA, synthetic | 5 |
| 27+0 ds | 5'-OHAAGCUGACCCUGAAGUUCAUCUGCACC-3' 3'-UUCGACUGGGACUUCAAGUAGACGUGGOH-5' | RNA, synthetic | |
| 27+2 s | 5'-OHGCUGACCCUGAAGUUCAUCUGCACCACUU-3' | RNA, synthetic | 6 |
| 27+2 a | 5'-OHGUGGUGCAGAUGAACUUCAGGGUCAGCUU-3' | RNA, synthetic | 7 |
| 27+2 ds | 5'-OHGCUGACCCUGAAGUUCAUCUGCACCACUU-3' 3'-UUCGACUGGGACUUCAAGUAGACGUGGUGOH-5' | RNA, synthetic | |
| 3pRNA | 5'-pppGGGGCUGACCCUGAAGUUCAUCUU-3' | RNA, in vitro transcribed | 2 |
| CpG-A | 5'-GGGGGACGATCGTCGGGGGG-3' | DNA | 3 |

| | | | |
|---|---|---|---|
| (p: monophosphate; ppp: triphosphate; under lined letters: phosphorothioate linkage 3' of the base; bold letters, CpG dinucleotides) | | | |

As shown in Figure 2B, regardless of the presence or the absence of a 5' triphosphate, all RNA oligonucleotides tested were capable of inducing IFN-α production from PDCs which primarily use TLR7 for short dsRNA recognition (see Example 1).

As shown in Figure 2B, whereas in vitro transcribed RNA oligonucleotide, 3pRNA, bearing 5' triphosphate, induced a significant amount of IFN-α, synthetic RNA oligonucleotides bearing 5' OH failed to induce any IFN-α production from monocytes, regardless whether the oligonucleotide had blunt ends or 3' overhangs.

These results indicate that 5' triphosphate is strictly required for IFN-α induction in monocytes. Since RNA recognition and IFN-α induction is primarily mediated by RIG-I in monocytes (see Example 1), this data suggest that blunt end is not recognized by RIG-I, at least not in the absence of 5' triphosphate.

### Example 3. Blunt end augments the immunostimulatory activity of synthetic double-stranded oligonucleotides bearing 5' triphosphate.

Since 3pRNA oligonucleotides are capable of inducing IFN-α production from PDCs via a TLR7-dependent pathway (see Example 1), in order to study RIG-I-dependent induction of IFN-α, RNA oligonucleotides (Figure 3 & Table 3) were transfected into purified monocytes, PDC-depleted PBMCs (PBMC-PDC) or chloroquine-treated PBMCs (PBMC+Chl).

The design and the designation of the RNA oligonucleotides are shown in Figure 3 and the sequences of the oligonucleotides are shown in Table 3.

**Table 3. RNA and DNA oligonucelotide sequences**

| Name | Sequence | 5' end | Type | SEQ ID NO |
|---|---|---|---|---|
| 3P-A | **A**ACACACACACACACACACACUUU | **3P** | RNA, syn | 8 |
| ivt3P-G | **G**ACACACACACACACACACACUUU | **3P** | RNA, ivt | 9 |
| 3P-G | **G**ACACACACACACACACACACUUU | **3P** | RNA, syn | 9 |
| 3P-C | **C**ACACACACACACACACACACUUU | **3P** | RNA, syn | 10 |
| 3P-U | **U**ACACACACACACACACACAC U U U | **3P** | RNA, syn | 11 |
| HO-A | **A**ACACACACACACACACACACUUU | OH | RNA, syn | 8 |
| P-A | **A**ACACACACACACACACACACUUU | P | RNA, syn | 8 |
| AS A26 | AAAGUGUGUGUGUGUGUGUGUGUUGU | OH | RNA, syn | 12 |
| AS A25 | AAAGUGUGUGUGUGUGUGUGUGUUG | OH | RNA, syn | 13 |
| AS A24+2A | AAAAAGUGUGUGUGUGUGUGUGUGUU | OH | RNA, syn | 14 |
| AS A24+A | AAAAGUGUGUGUGUGUGUGUGUGUU | OH | RNA, syn | 15 |
| AS A24 | AAAGUGUGUGUGUGUGUGUGUGUU | OH | RNA, syn | 16 |
| AS A23 | AAGUGUGUGUGUGUGUGUGUGUU | OH | RNA, syn | 17 |
| AS A21 | GUGUGUGUGUGUGUGUGUGUU | OH | RNA, syn | 18 |
| AS A20 | UGUGUGUGUGUGUGUGUGUU | OH | RNA, syn | 19 |
| AS A19 | GUGUGUGUGUGUGUGUGUU | OH | RNA, syn | 20 |
| AS A17 | GUGUGUGUGUGUGUGUU | OH | RNA, syn | 21 |
| AS A15 | GUGUGUGUGUGUGUU | OH | RNA, syn | 22 |
| AS A13 | GUGUGUGUGUGUU | OH | RNA, syn | 23 |
| AS G26 | AAAGUGUGUGUGUGUGUGUGUGUCGU | OH | RNA, syn | 24 |
| AS G25 | AAAGUGUGUGUGUGUGUGUGUGUCG | OH | RNA, syn | 25 |
| AS G24+2A | AAAAAGUGUGUGUGUGUGUGUGUGUC | OH | RNA, syn | 26 |
| AS G24+A | AAAAGUGUGUGUGUGUGUGUGUGUC | OH | RNA, syn | 27 |
| AS G24 | AAAGUGUGUGUGUGUGUGUGUGUC | OH | RNA, syn | 28 |
| AS G23 | AAGUGUGUGUGUGUGUGUGUGUC | OH | RNA, syn | 29 |
| AS G21 | GUGUGUGUGUGUGUGUGUGUC | OH | RNA, syn | 30 |
| AS G20 | UGUGUGUGUGUGUGUGUGUC | OH | RNA, syn | 31 |
| AS G19 | GUGUGUGUGUGUGUGUGUC | OH | RNA, syn | 32 |
| AS G17 | GUGUGUGUGUGUGUGUC | OH | RNA, syn | 33 |
| AS G15 | GUGUGUGUGUGUGUC | OH | RNA, syn | 34 |
| AS G13 | GUGUGUGUGUGUC | OH | RNA, syn | 35 |
| AS C26 | AAAGUGUGUGUGUGUGUGUGUGUGGU | OH | RNA, syn | 36 |
| AS C24 | AAAGUGUGUGUGUGUGUGUGUGUG | OH | RNA, syn | 37 |
| AS U26 | AAAGUGUGUGUGUGUGUGUGUGUAGU | OH | RNA, syn | 38 |
| AS U24 | AAAGUGUGUGUGUGUGUGUGUGUA | OH | RNA, syn | 39 |
| AS23 | AAAGUGUGUGUGUGUGUGUGUGU | OH | RNA, syn | 40 |
| AS21 | AAAGUGUGUGUGUGUGUGUGU | OH | RNA, syn | 41 |
| AS19 | AAAGUGUGUGUGUGUGUGU | OH | RNA, syn | 42 |
| IVT2 | GACGACGACGACGACGACGACGACGACGAC | 3P | RNA, ivt | 43 |
| dAdT | (AT)₂₀₀₋₄₀₀₀ | P | DNA, syn | |

| | | | | |
|---|---|---|---|---|
| (syn: synthetic; ivt: in vitro transcribed) | | | | |

As shown in Figures 4A, 5A and 6A, a minimal length of 21 nucleotides was required for a double-stranded 3pRNA oligonucleotide to induce IFN-α from monocytes. Furthermore, the highest IFN-α-inducing activity was seen when the double-stranded 3pRNA oligonucleotide had a blunt end at the same end bearing the 5' triphosphate. The structure of the end not bearing a 5' triphosphate did not appear to affect the IFN-α-inducing activity of the oligonucleotide to any significant degree. Moreover, dsRNA oligonucleotides with an A at the 5' end bearing the 5' triphosphate were more potent in inducing IFN-α than those with a G or U at the same position; dsRNA oligonucleotides with a C at the 5' end bearing the 5' triphosphate was the least potent. Similar results were obtained with PDC-depleted PBMCs and chloroquine-treated PBMCs (Figure 4-6, B & C).

In contrast, double-stranded oligonucleotides bearing a 5' monophosphate were not effective at inducing IFN-α, regardless of the end structures (Figure 6).

These data suggest 5' triphosphate is required for RIG-I recognition. Furthermore, blunt end is a molecular signature recognized by RIG-I; it augments the potency of a synthetic double-stranded RNA oligonucleotide bearing 5' triphosphate at the same end in inducing IFN-α via the RIG-I pathway.

### Example 4. Single-stranded RNA transcripts can be generated by in vitro transcription.

### (Comparative example)

Both synthetic dsRNA bearing 5' triphosphate and in vitro transcribed ssRNA have been shown to be able to activate RIG-I ^{15, 25}. The present inventors found that double-stranded configuration is required for RIG-I activation ¹⁹. It was hypothesized that ssRNA obtained by in vitro transcription was capable of activating RIG-I probably due to the presence of aberrant RNA transcripts which had a double-stranded configuration.

Indeed, when ssRNA was transcribed in vitro in the presence of all 4 NTP's (i.e., ATP, UTP, GTP, CTP) and run on a urea polyacrylamide gel, two bands were observed, indicating the presence double-stranded species (Figure 7C, sample 4, "ivt3P-G_ACA").

**Table 4. Sequence of RNA oligonucleotides.**

| name | sequence | type | SEQ ID NO |
|---|---|---|---|
| 3P-G | 5'-pppGACACACACACACACACACACUUU-3' | RNA, synthetic | 9 |
| AS-G21 | 5'-OHGUGUGUGUGUGUGUGUGUGUC-3' | RNA, synthetic | 30 |
| inv3P-G_ACA | 5'-pppGACACACACACACACACACACACA-3' | RNA, in vitro transcribed | 44 |

Subsequently, *in vitro* transcription was carried out in the absence of UTP. Surprisingly, the upper band disappeared from the gel and the transcript did not induce any IFN-a induction from purified primary human monocytes (Figure 7A-C, sample 5, "ivt3P-G_ACA-U").

The addition of a synthetic complementary strand (AS G21) to such an in vitro transcribed single-strand RNA oligonucleotide (ivt3P-G_ACA-U ) which by itsself has no IFN-inducing activity restored the IFN-inducing activity (Figure 1A-C, sample 6, "ivt3P-G_ACA-U + AS G21").

This result suggests that it is possible to obtain an ssRNA without double strand formation and without IFN-inducing activity from in vitro transcription. Such an in vitro transcribed ssRNA can become an active RIG-I ligand only upon double strand formation with a complementary strand.

### REFERENCES

- 1.: Kawai T, Akira S. Nat Immunol 2006;7(2):131-7.
- 2.: Alexopoulou L et al. Nature 2001;413(6857):732-8.
- 3.: Diebold SS et al. Science 2004;303(5663):1529-31.
- 4.: Heil F et al. Science 2004;303(5663):1526-9.
- 5.: Hornung V et al. Nat Med 2005;11(3):263-70.
- 6.: Hemmi H et al. Nature 2000;408(6813):740-5.
- 7.: Matsumoto M et al. J Immunol 2003;171(6):3154-62.
- 8.: Nishiya T, DeFranco AL. J Biol Chem 2004;279(18):19008-17.
- 9.: Latz E et al. Nat Immunol 2004;5(2):190-8.
- 10.: Kato H et al. Nature 2006;441(7089):101-5.
- 11.: Yoneyama M et al. Nat Immunol 2004;5(7):730-7.
- 12.: Kato H et al. Immunity 2005;23(1):19-28.
- 13.: Lau CM et al. J Exp Med 2005;202(9):1171-7.
- 14.: Gitlin L et al. Proc Natl Acad Sci U S A 2006;103(22):8459-64.
- 15.: Hornung V et al. Science 2006;314(5801):994-7.
- 16.: Marques JT et al. Nat Biotechnol 2006;24(5):559-65.
- 17.: Judge AD et al. Nat Biotechnol 2005;23(4):457-62.
- 18.: Sioud M. Eur J Immunol 2006;36(5):1222-30.
- 19.: WO 2008/017473
- 20.: Kim DH et al. Nat Biotechnol 2004;22:321-5.
- 21.: US 2006/0178334
- 22.: WO 2003/086280
- 23.: Reynold et al. Nat Biotechnol 2004; 22:326-30.
- 24.: Takahasi K et al. Molecular Cell 2008; 29:1-13.
- 25.: Pichlmair A et al. Science 2006; 314:997-1001.
- 26.: Cui S et al. Molecular Cell 2008; 29:169-179.
- 27.: Ludwig J & Eckstein F J Org Chem 1989; 54:631-635.
- 28.: WO 2007/031319
- 29.: WO 2007/031322
- 30.: Gondai T et al. Nucleic Acids Res 36(3):e18
- 31.: Fruscoloni et al. Proceedings of the national academy of sciences 2003; 100(4):1639-1644

## Claims

1. An essentially homogenous population of a chemically synthesized oligonucleotide,
which is capable of activating RIG-I and inducing a type I IFN response,
wherein said essentially homogenous population means that at least 85 % of the oligonucleotides have the same chemical identity, including the same nucleotide sequence, backbone, modifications, length, and end structures,
wherein the oligonucleotide has two blunt ends,
wherein the blunt ends are ends of a fully double-stranded section, and wherein the fully double-stranded section is at least 21 and at most 30 base pairs in length, and
the double-stranded oligonucleotide has one blunt end bearing a 5' triphosphate attached to the most 5' ribonucleotide and a second blunt end which does not bear a 5' triphosphate,
wherein the most 5' ribonucleotide with the triphosphate attached to is G, U or C,
wherein the oligonucleotide comprises at least 1, preferably at least 3, more preferably at least 6 ribonucleotide(s) at the 5' end at the blunt end bearing the 5' triphosphate, and
wherein the oligonucleotide is free of modifications selected from the group consisting of pseudouridine, 2-thiouridine, 2'Fluorine-dNTP and 2-O-methylated NTP, or wherein the oligonucleotide contains modified nucleotides that do not compromise the type I IFN-inducing activity,
wherein the 5' triphosphate is free of any cap structure.

2. The oligonucleotide population of claim 1, wherein said essentially homogenous population means that at least 90 %, preferably at least 95%, more preferably at least 95%, more preferably at least 97%, and most preferably at least 98% of the oligonucleotides have the same chemical identity, including the same nucleotide sequence, backbone, modifications, length, and end structures.

3. The oligonucleotide population of any one of claims 1 or 2, wherein the fully double-stranded section is at least 22 base pairs in length, more preferably 23 base pairs in length, and most preferably 24 base pairs in length.

4. The oligonucleotide population of any one of claims 1-3, wherein the most 5' ribonucleotide with the triphosphate attached to is G.

5. The oligonucleotide population of any one of claims 1-4, wherein fully double-stranded section is composed solely of ribonucleotides.

6. The oligonucleotide population of any one of claims 1-5, wherein the sequence of the first 3 ribonucleotides following the most 5' ribonucleotide bearing the 5' triphosphate is selected from AGU, AAG, UGG, UUA, ACG, UGA, GUU, UUG, ACA, GAA, GCA, ACU, UCG, GGA, UCA, UGC, GUA, ACC, GGU, AAC, UGU, CUG, CGA, CAG, AGG, CAU, CGC, AAU, CGG, UUC, GUG, CAA, UCC, wherein the sequence is in the 5'->3' direction.

7. The oligonucleotide population of any one of claims 1-6, wherein the oligonucleotide comprises at least one inosine.

8. The oligonucleotide population of any one of claims 1-7, wherein the oligonucleotide has gene-silencing activity.

9. A pharmaceutical composition comprising at least one oligonucleotide population according to any one of claims 1-8, and a pharmaceutically acceptable carrier.

10. The pharmaceutical composition of claim 9, further comprising at least one agent selected from an immunostimulatory agent, an anti-viral agent, an anti-bacterial agent, an anti-tumor agent, retinoic acid, IFN-o, and IFN-β.

11. A pharmaceutical composition according to any one of claims 9 or 10 for use in the prevention and/or treatment of a disease or condition selected from an infection, a tumor, and an immune disorder.

12. The pharmaceutical composition for use of claim 11, wherein the pharmaceutical composition is for administration in combination with at least one treatment selected from a prophylactic and/or a therapeutic treatment of an infection, a tumor, and an immune disorder.

13. An in vitro method for inducing type I IFN production in a cell, comprising the steps of:
(a) mixing at least one oligonucleotide population of any of claims 1-8 with a complexation agent; and
(b) contacting a cell with the mixture of (a), wherein the cell expresses RIG-I.

## Patentansprüche

1. Eine im Wesentlichen homogene Population eines chemisch synthetisierten Oligonukleotids,
das in der Lage ist, RIG-I zu aktivieren und eine Typ-I-IFN-Reaktion auszulösen,
wobei die im Wesentlichen homogene Population bedeutet, dass mindestens 85 % der Oligonukleotide die gleiche chemische Identität aufweisen, einschließlich der gleichen Nukleotidsequenz, des Rückgrats, der Modifikationen, der Länge und der Endstrukturen,
wobei das Oligonukleotid zwei glatte Enden aufweist,
wobei die glatten Enden Enden eines vollständig doppelsträngigen Abschnitts sind, und wobei der vollständig doppelsträngige Abschnitt mindestens 21 und höchstens 30 Basenpaare lang ist, und
das doppelsträngige Oligonukleotid ein glattes Ende aufweist, das ein 5'-Triphosphat trägt, das an das 5'-Ribonukleotid gebunden ist, und ein zweites glattes Ende, das kein 5'-Triphosphat trägt,
wobei das 5'-Ribonukleotid, an das das Triphosphat gebunden ist, G, U oder C ist,
wobei das Oligonukleotid mindestens 1, vorzugsweise mindestens 3, noch bevorzugter mindestens 6 Ribonukleotid(e) am 5'-Ende an dem glatten Ende, welches das 5'-Triphosphat trägt, umfasst, und
wobei das Oligonukleotid frei von Modifikationen ist, die ausgewählt sind aus der Gruppe bestehend aus Pseudouridin, 2-Thiouridin, 2'Fluor-dNTP und 2-O-methyliertem NTP, oder wobei das Oligonukleotid modifizierte Nukleotide enthält, die die Typ I IFNinduzierende Aktivität nicht beeinträchtigen,
wobei das 5'-Triphosphat frei von jeglicher Kappenstruktur ist.

2. Oligonukleotidpopulation nach Anspruch 1, wobei die im Wesentlichen homogene Population bedeutet, dass mindestens 90 %, vorzugsweise mindestens 95 %, noch bevorzugter mindestens 95 %, noch bevorzugter mindestens 97 % und am meisten bevorzugt mindestens 98 % der Oligonukleotide die gleiche chemische Identität aufweisen, einschließlich der gleichen Nukleotidsequenz, des Rückgrats, der Modifikationen, der Länge und der Endstrukturen.

3. Oligonukleotidpopulation nach einem beliebigen der Ansprüche 1 oder 2, wobei der vollständig doppelsträngige Abschnitt mindestens 22 Basenpaare, vorzugsweise 23 Basenpaare und besonders bevorzugt 24 Basenpaare lang ist.

4. Oligonukleotidpopulation nach einem beliebigen der Ansprüche 1 bis 3, wobei das 5'-Ribonukleotid, an das das Triphosphat gebunden ist, G ist.

5. Oligonukleotidpopulation nach einem beliebigen der Ansprüche 1-4, wobei der vollständig doppelsträngige Abschnitt ausschließlich aus Ribonukleotiden besteht.

6. Oligonukleotidpopulation nach einem beliebigen der Ansprüche 1-5, wobei die Sequenz der ersten 3 Ribonukleotide, die auf das 5'-Ribonukleotid folgen, das das 5'-Triphosphat trägt, ausgewählt ist aus AGU, AAG, UGG, UUA, ACG, UGA, GUU, UUG, ACA, GAA, GCA, ACU, UCG, GGA, UCA, UGC, GUA, ACC, GGU, AAC, UGU, CUG, CGA, CAG, AGG, CAU, CGC, AAU, CGG, UUC, GUG, CAA, UCC, wobei die Sequenz in der 5'->3'-Richtung verläuft.

7. Oligonukleotidpopulation nach einem beliebigen der Ansprüche 1 bis 6, wobei das Oligonukleotid mindestens ein Inosin umfasst.

8. Oligonukleotidpopulation nach einem beliebigen der Ansprüche 1-7, wobei das Oligonukleotid eine Gen-Stilllegungs-Aktivität aufweist.

9. Pharmazeutische Zusammensetzung, die mindestens eine Oligonukleotidpopulation nach einem der Ansprüche 1-8 und einen pharmazeutisch verträglichen Träger umfasst.

10. Pharmazeutische Zusammensetzung nach Anspruch 9 , weiterhin umfassend mindestens ein Mittel, das ausgewählt ist aus einem immunstimulierenden Mittel, einem antiviralen Mittel, einem antibakteriellen Mittel, einem Antitumormittel, Retinsäure, IFN-o und IFN-β.

11. Pharmazeutische Zusammensetzung nach einem der Ansprüche 9 oder 10 zur Verwendung bei der Vorbeugung und/oder Behandlung einer Krankheit oder eines Zustands, der ausgewählt ist aus einer Infektion, einem Tumor und einer Immunstörung.

12. Pharmazeutische Zusammensetzung zur Verwendung nach Anspruch 11, wobei die pharmazeutische Zusammensetzung zur Verabreichung in Kombination mit mindestens einer Behandlung, die ausgewählt ist aus einer prophylaktischen und/oder therapeutischen Behandlung einer Infektion, eines Tumors und einer Immunstörung, bestimmt ist.

13. In-vitro-Verfahren zum Induzieren der Typ-I-IFN-Produktion in einer Zelle, umfassend die folgenden Schritte:
(a) Mischen mindestens einer Oligonukleotidpopulation nach einem beliebigen der Ansprüche 1-8 mit einem Komplexierungsmittel; und
(b) Inkontaktbringen einer Zelle mit der Mischung aus (a), wobei die Zelle RIG-I exprimiert.

## Revendications

1. Population essentiellement homogène d'un oligonucléotide synthétisé chimiquement, qui est apte à activer RIG-I et à induire une réponse IFN de type I,
dans laquelle ladite population essentiellement homogène signifie qu'au moins 85 % des oligonucléotides ont la même identité chimique, y compris la même séquence nucléotidique, le même squelette, les mêmes modifications, longueur et structures d'extrémité,
dans laquelle l'oligonucléotide a deux extrémités franches,
dans laquelle les extrémités franches sont les extrémités d'une section entièrement double brin, et dans laquelle la section entièrement double brin a une longueur d'au moins 21 et d'au plus 30 paires de bases, et
l'oligonucléotide double brin a une extrémité franche portant un triphosphate en 5' lié au ribonucléotide le plus en 5' et une seconde extrémité franche qui ne porte pas de triphosphate en 5',
dans laquelle le ribonucléotide le plus en 5' avec le triphosphate lié est G, U ou C,
dans laquelle l'oligonucléotide comprend au moins 1, de préférence au moins 3, plus préférablement au moins 6 ribonucléotide(s) à l'extrémité 5' au niveau de l'extrémité franche portant le triphosphate en 5', et
dans laquelle l'oligonucléotide est dépourvu de modifications choisies dans le groupe constitué de la pseudouridine, la 2-thiouridine, un 2'fluor-dNTP et un NTP 2-O-méthylé, ou dans laquelle l'oligonucléotide contient des nucléotides modifiés qui ne compromettent pas l'activité d'induction de l'IFN de type I,
dans laquelle le triphosphate en 5' est dépourvu de toute structure de coiffe.

2. Population d'oligonucléotides selon la revendication 1, dans laquelle ladite population essentiellement homogène signifie qu'au moins 90 %, de préférence au moins 95 %, plus préférablement au moins 95 %, plus préférablement au moins 97 %, et de manière préférée entre toutes au moins 98 % des oligonucléotides ont la même identité chimique, y compris la même séquence nucléotidique, le même squelette, les mêmes modifications, longueur et structures d'extrémité.

3. Population d'oligonucléotides selon l'une quelconque des revendications 1 ou 2, dans laquelle la section entièrement double brin a au moins une longueur de 22 paires de bases, plus préférablement une longueur de 23 paires de bases, et de manière préférée entre toutes une longueur de 24 paires de bases.

4. Population d'oligonucléotides selon l'une quelconque des revendications 1 à 3, dans laquelle le ribonucléotide le plus en 5' auquel est lié le triphosphate est G.

5. Population d'oligonucléotides selon l'une quelconque des revendications 1 à 4, dans laquelle la section entièrement double brin est composée uniquement de ribonucléotides.

6. Population d'oligonucléotides selon l'une quelconque des revendications 1 à 5, dans laquelle la séquence des 3 premiers ribonucléotides suivant le ribonucléotide le plus en 5' portant le triphosphate en 5' est choisie parmi AGU, AAG, UGG, UUA, ACG, UGA, GUU, UUG, ACA, GAA, GCA, ACU, UCG, GGA, UCA, UGC, GUA, ACC, GGU, AAC, UGU, CUG, CGA, CAG, AGG, CAU, CGC, AAU, CGG, UUC, GUG, CAA, UCC, dans laquelle la séquence est dans le sens 5'->3'.

7. Population d'oligonucléotides selon l'une quelconque des revendications 1 à 6, dans laquelle l'oligonucléotide comprend au moins une inosine.

8. Population d'oligonucléotides selon l'une quelconque des revendications 1 à 7, dans laquelle l'oligonucléotide présente une activité de silençage génique.

9. Composition pharmaceutique comprenant au moins une population d'oligonucléotides selon l'une quelconque des revendications 1 à 8, et un support pharmaceutiquement acceptable.

10. Composition pharmaceutique selon la revendication 9, comprenant en outre au moins un agent choisi parmi un agent immunostimulant, un agent antiviral, un agent antibactérien, un agent anti-tumoral, l'acide rétinoïque, l'IFN-a et l'IFN-β.

11. Composition pharmaceutique selon l'une quelconque des revendications 9 ou 10 pour une utilisation dans la prévention et/ou le traitement d'une maladie ou d'un état choisi parmi une infection, une tumeur et un trouble immunitaire.

12. Composition pharmaceutique pour une utilisation selon la revendication 11, dans laquelle la composition pharmaceutique est destinée à être administrée en association avec au moins un traitement choisi parmi un traitement prophylactique et/ou thérapeutique d'une infection, d'une tumeur et d'un trouble immunitaire.

13. Procédé in vitro d'induction de la production d'IFN de type I dans une cellule, comprenant les étapes de :
(a) mélange d'au moins une population d'oligonucléotides selon l'une quelconque des revendications 1 à 8 avec un agent de complexation ; et
(b) mise en contact d'une cellule avec le mélange de (a), dans laquelle la cellule exprime RIG-I.
